# EUROPEAN PATENT APPLICATION

(11) **EP 1 002 551 A2**
(43) Date of publication of application: **24.05.2000**
(21) Application number: 99107531.8
(22) Date of filing: 15.04.1999
(51) Int. Cl.: A61M 5/315

(54) **Easy-slip plunger/plunger rod assembly for a syringe or a cartridge**

(30) Priority: 19.11.1998 US 196085
(71) Applicant: Bracco International B.V., 1077 ZX Amsterdam (NL)
(72) Inventor: Gabbard, Mark E., Delmar De 19940 (US); Robinson, Michelle L., Newton, PA 18940 (US); Gabbard, Timothy J., Salisbury, MD 21804 (US); Niedospial, John J. Jr., Burlington, NJ 08016 (US)
(74) Representative: Gervasi, Gemma, Dr.

(57) **Abstract**

It is disclosed an improved elastomeric plunger ring (298, 356, 372, 382, 398), provided for use in conjunction with a cartridge or syringe (284), which allows a low-friction, slideable engagement between the elastomeric plunger ring and the inside wall (288) of a syringe or a cartridge barrel (286), said low-friction, slideable sealing engagement requiring reduced breakaway and running forces for operation of the cartridge or syringe (284).

## Description

### Field of the Invention

This invention relates to plunger rod and plunger combinations utilized in syringes and cartridges for the introduction or withdrawal of fluids to and from a patient. More particularly, the invention relates to glass or plastic syringes and cartridges which may be pre-filled with a pharmaceutical fluid for injections to a patient.

### Background of the invention

Syringes and cartridges made of glass or polymeric material for delivery of fluids to and from a patient have been proposed and utilized by the prior art, and have achieved a highly developed state. Various requirements related to specific delivery systems have also been addressed. While specific requirements of fluid delivery to and from a patient may vary, means of delivery remain essentially the same and may be characterized by the following general description of a syringe.

A syringe comprises:
a) a cylindrical barrel having a proximal end designed for receiving a plunger with or without a plunger rod removably attached to the plunger or being integral with the plunger, and a distal end adapted to mount a needle or luer connector thereon; and
b) a plunger slideably mounted in the barrel.

The plunger is inserted into the barrel at the proximal end of the syringe and thus when fluid is contained in the barrel it may be expelled by pushing the plunger in the barrel towards its distal end; or when the syringe is used to withdraw fluid from a patient, the plunger located at the distal end of the barrel is pulled towards the proximal end of the syringe thereby drawing fluid into the barrel. Since a fluid-tight seal is necessary between the plunger and the inside wall of the barrel, a resilient rubber tip is positioned on the distal end of the plunger, or typically, the plunger is made of resilient rubber-like material. In some of the syringes of the prior art the rubber tip has been replaced with a generally flat, circular disk as part of the plunger.

In order to assure air-tight seal between the inside wall of the syringe barrel and the plunger, prior art plungers are manufactured with a larger outside diameter than the inside diameter of the syringe barrels. When the plunger is introduced into the syringe barrel, it is sufficiently compressed to provide adequate pressure between the inside wall of the syringe and the plunger to seal the interface and withstand the challenges of filling, injecting and withdrawing fluids using the syringe without leakage.

In addition to a leakage-proof seal, another requirement in the syringe/plunger combination is the chemical stability of both the syringe and the plunger. While syringes being made of glass or thermoplastic materials are sufficiently chemically inert to pharmaceutical and biological fluids contained therein, the plungers made of natural rubber or butyl rubber have some undesirable properties. The rubber contains additional chemical components such as fillers and accelerators introduced during the curing process which tend to exude to the surface of the plunger during the contact between the plunger and the fluid contained in the syringe. Such exudate is undesirable in an injection or when a biological fluid, such as blood, is withdrawn from a patient for testing purposes. The problem is further aggravated when there is a long-term storage of the content of the pharmaceutical /biological fluid in the syringe. Recognizing the problem of contamination caused by exudates from plungers made of rubber, the prior art has provided plungers made of thermoplastic materials which do not contain the additives that rubber plungers contain. However, thermoplastic materials are not as resilient as rubbers and the seals formed between thermoplastic plungers and the inside walls of syringes tend to be inadequate in some circumstances. Also, over a period of time on storage the thermoplastic plunger may achieve a compression stage wherein the outside diameter of the plunger is reduced thereby no longer capable of forming a tight seal between it and the inside wall of the syringe.

In addition to the tendency of leakage, the thermoplastic plunger does not slide smoothly in the syringe barrel and requires the exertion of excessive force on the plunger rod to move the plunger in the barrel. The exertion of excessive force on the plunger rod may result in uneven delivery of the fluid to the patient or insertion of the needle into a vein or tissue area to an undesirable depth.

In both the rubber and thermoplastic plungers a relatively large compressive force must be exerted on the plungers by the syringe barrel to provide for a tight, leakproof seal. This quality of the seal, however, makes the movement of the plunger difficult. To remedy the problem the prior art used lubricants to reduce friction and drag between the plunger and the inside surface of the syringe barrel. The use of such lubricants, however, is also undesirable with certain parenteral fluids which tend to disperse or dissolve in the parenteral fluids thereby contaminating the parenteral fluids. Attempts to avoid the use of lubricants included the use of various plunger configurations, such as plungers that were provided with one or more ribs projecting forwardly or rearwardly in the barrel to reduce the frictional drag between the plunger and the inside surface of the barrel.

As specific documents of the state of the art, some examples are the following:
■ US patent No. 4,543,093 relates to a variable sealing pressure plunger rod assembly comprising a plunger rod and a flexible thermoplastic stopper; the thermoplastic stopper including an inside cavity to receive the distal end portion of the plunger rod;
■ US patent No. 4,673,396 relates to a syringe cartridge used in conjunction with blood gas measurements; the syringe cartridge contains a plunger piston 14 having a dome 62; a pair of seal rings 65 on the exterior of the plunger piston are provided to engage the interior wall of the cartridge;
■ US patent No. 5,314,416 discloses a low friction syringe assembly comprising: a plunger rod and a plunger tip which are designed to provide a low friction resistance against the interior surface of the syringe barrel while the syringe barrel is at rest but which will increase the sealing pressure between the plunger tip and the interior surface of the syringe barrel in proportion to the amount of force applied to the plunger rod;
■ US patent No. 5,413,563 pertains to a pre-filled syringe having a plunger, plunger insert and plunger rod; the plunger insert 68 exerts force against bottom rim 39 of plunger 30 during manual aspiration;
■ US patent No. 5,624,405 relates to a pre-filled syringe and syringe tip assembly comprising: a plunger 6, plunger rod 62 and a gasket 61 fitted on the top of the plunger rod 62 so as to move smoothly along the lumen of the syringe body 1.
All of the above-cited references use a plunger rod and a plunger assembly in the barrel of a syringe cartridge or vial and all are, at least in part, directed to a leak-proof seal and easy sliding objective. While fluid tightness and sliding property have improved with these attempts, it appears that improvement in one of these properties is not quite achieved without corresponding decrease in the other property: increasing fluid tightness tends to result in decreasing sliding property, while increasing sliding property tends to result in decreasing fluid tightness. None of the references cited disclose or suggest combination of plunger rod and plunger having the desirable characteristics disclosed in the present invention.

The present invention addresses the following main requirements in a syringe or cartridge, using the elastomeric plunger according to the invention:
reduction of breakaway forces;
reduction of running forces;
insuring against leakage; and
no siliconization to achieve the above.

These and other related requirements will be addressed in the embodiments provided by the present invention the description of which follows.

### SUMMARY OF THE INVENTION

The present invention discloses a new and improved elastomeric plunger ring, provided for use in conjunction with a syringe or cartridge, which allows a low-friction, slideable engagement between the elastomeric plunger ring and the inside wall of a syringe or a cartridge barrel, said low-friction, slideable sealing engagement requiring reduced breakaway and running forces for operation of the syringe or cartridge.
Accordingly, a first aspect of the invention relates to an elastomeric plunger ring designed to provide a low-friction slideable sealing engagement between the elastomeric plunger ring and the inside wall of a syringe or cartridge barrel, said elastomeric plunger ring effectively interfacing and conforming to dimensional and geometric irregularities in said barrel, said low-friction slideable sealing engagement requiring reduced breakaway and running forces for operation of the syringe or the cartridge, the plunger ring comprising:
a top conical or flat portion for interfacing a fluid contained in said syringe or cartridge barrel;
a cylindrical side portion having an inside wall and an outside wall;
a plurality of rims projecting distally, proximally or horizontally from said outside wall of said cylindrical side portion for interfacing the inside wall of said syringe or cartridge barrel and to form the low-friction slideable sealing engagement therewith; and means on the inside wall of said plunger ring to receive a thermoplastic, non-resilient support means to form a non-slideable sealing engagement therewith, wherein said thermoplastic, non-resilient support means is a plunger rod tip or a plunger rod tip integral with a plunger rod.

The plunger ring according to the invention is a full-face plunger ring of various configuration designed to completely seal a plunger rod tip and to interface the content of a syringe or cartridge barrel without allowing contact between the plunger rod tip and the content of the barrel of the syringe or cartridge. This disclosure in intended for use with fluids which do not substantially interact with the elastomeric material of the plunger ring. The term "full-face" as use herein has the meaning of interfacing the fluid contained in the barrel of a syringe or cartridge without allowing interfacing of the fluid with the plunger rod tip made of thermoplastic materials. The plunger rod tip may be separate from the plunger rod or it may be integral therewith.

According to a further aspect, the present invention relates to a syringe designed for injecting a fluid into a site or withdrawing a fluid from a site comprising:
(a) a syringe barrel having an inside wall defining a cylindrical chamber for retaining a fluid therein; said syringe barrel having:
   (1) a distal end terminating in a tapered tip and having a bore therethrough to which an injection needle or a connector equipped with a tubing conduit can be attached; and
   (2) a proximal end for receiving an elastomeric plunger ring;
(b) the elastomeric plunger ring according to the invention.

The syringe barrel can be made of glass or polymeric material.
According to another further aspect, the invention relates to a cartridge, optionally prefilled, designed for injecting a fluid into a site comprising:
(a) a cartridge barrel having an inside wall defining a cylindrical chamber for retaining a fluid therein; said cartridge barrel having:
   (1) a distal end terminating in a tapered tip and having a bore therethrough to which an injection needle or a connector equipped with a tubing conduit can be attached; and
   (2) a proximal end for receiving an elastomeric plunger ring;
(b) the elastomeric plunger ring according to the invention.
The cartridge barrel can be made of glass or of polymeric material.
The fluid comprised in the cartridge can be an injectable medical fluid, preferably a diagnostic contrast media fluid.

### BRIEF DESCRIPTION OF THE DRAWINGS

- FIG. 1: is a perspective view of the assembled syringe containing a plunger ring, plunger rod and plunger rod tip according to the present invention;
- FIG. 2: is a longitudinal cross-section of the plunger rod removed from the syringe taken along the line 2-2 of FIG. 1;
- FIG. 3A: is a perspective view of the plunger ring shown in FIG. 1;
- FIG. 3B: is a side elevational view of the plunger ring shown in FIG. 3A;
- FIG. 4A: shows a longitudinal cross-section of the plunger rod tip with plunger ring thereon, wherein the plunger rod tip has internal female threads for attachment to a male-threaded plunger rod;
- FIG. 4B: shows a top plan view of the plunger rod tip with plunger ring thereon shown in FIG. 4A;
- FIG. 5A: shows the longitudinal cross-section of another plunger rod tip designed to carry one internal seal plunger ring thereon wherein the plunger rod tip has internal female threads for attachment to a male-threaded plunger rod;
- FIG. 5B: shows a top plan view of the plunger rod tip shown in FIG. 5A;
- FIG. 6A: shows the longitudinal cross-section of still another plunger rod tip, with accentuated contours, designed to carry one internal seal plunger ring thereon, wherein the plunger rod tip has internal female threads for attachment to a male-threaded plunger rod;
- FIG. 6B: shows a top plan view of the plunger rod tip shown in FIG. 6A;
- FIG. 7A: shows the longitudinal cross-section of still another plunger rod tip designed to carry two internal seals plunger ring thereon, wherein the plunger rod tip has internal female threads for attachment to a male-threaded plunger rod;
- FIG. 7B: shows a top pian view of the plunger rod tip shown in FIG. 7A;
- FIG. 8: shows the longitudinal cross-section of a plunger ring having one internal seal;
- FIG. 9: shows the longitudinal cross-section of the plunger ring having one internal seal shown in FIG. 8 and the direction of various pressures exerted on the plunger ring in use;
- FIG. 10A: shows the longitudinal cross-section of still another plunger ring having one internal seal in place on the plunger rod tip;
- FIG. 10B: shows a side elevational view of the cross-section of the plunger ring in place on the plunger rod tip shown in FIG. 10A;
- FIG. 11A: shows the longitudinal cross-section of still another plunger ring having one internal seal in place on the plunger rod tip and is designed to provide leak-proof protection in one direction;
- FIG. 11B: shows a side elevational view of the cross-section of the plunger ring in place on the plunger rod tip shown in FIG. 11A;
- FIG. 12A: shows the longitudinal cross-section of still another plunger ring having one internal seal and is designed to provide leak-proof protection in both directions;
- FIG. 12B: shows a side elevational view of the cross-section of the plunger ring in place on the plunger rod tip shown in FIG. 12A;
- FIG. 13A: shows the longitudinal cross-section of still another plunger ring in place on the plunger rod tip having one internal seal and is designed to provide leak-proof protection in both directions;
- FIG. 13B: shows a side elevational view of the cross-section of the plunger ring in place on the plunger rod tip shown in FIG. 13A;
- FIG. 14: is a longitudinal fragmentary cross-section of a typical prior art syringe, plunger and plunger rod;
- FIG. 15: is an enlarged fragmentary cross-section of the prior art plunger and plunge rod of FIG. 14;
- FIG. 16: shows a graph of breakaway forces between the inside wall of the barrel and a butyl rubber plunger with silicone of the prior art;
- FIG. 17: shows a graph of breakaway forces between the inside wall of the barrel and the butyl rubber plunger without silicone of the prior art;
- FIG. 18: shows a graph of running forces between the inside wall of the barrel and the butyl rubber plunger without silicone of the prior art;
- FIG. 19: shows a graph of running forces between the inside wall of the barrel and the butyl rubber plunger with silicone of the prior art;
- FIG. 20: shows a graph of average breakaway forces between the inside wall of the barrel and butyl rubber plunger with silicone of the prior art and the average breakaway forces between the inside wall of the barrel and the plunger rod tip/plunger ring combination with silicone of the present invention;
- FIG. 21: shows a graph of average breakaway forces between the inside wall of the barrel and butyl rubber plunger without silicone of the prior art and the average breakaway forces between the inside wall of the barrel and the plunger rod tip/plunger ring combination without silicone, wherein the plunger ring is made of Silicone(50), Nitrial(65), Nitrial(45), Viton(65) or Butyl(52) rubbers;
- FIG. 22: shows a graph of running forces between the inside wall of the barrel and the plunger rod tip/plunger ring combination with silicone of the present invention;
- FIG. 23: shows a graph of running forces between the inside wall of the barrel and the plunger of the prior art made of butyl rubber with silicone, versus a graph of running forces between the inside wall of the barrel and the plunger rod tip/plunger ring combination made of butyl rubber with silicone of the present invention;
- FIG. 24: shows a graph of running forces between the inside wall of the barrel and the plunger made of butyl rubber without silicone of the prior art;
- FIG. 25: shows a graph of running forces between the inside wall of the barrel and the plunger rod tip/plunger ring combination without silicone of the present invention; and
- FIG. 26: shows a graph of running forces between the inside wall of a barrel and the plunger of the prior art made of butyl rubber without silicone, versus a graph of running forces between the inside wall of the barrel and the plunger rod tip/plunger ring combination made of butyl rubber without silicone.
- FIG. 27: is a perspective view of an assembled syringe containing a hill-face plunger ring, a plunger rod and plunger rod tip according to another embodiment of the present invention;
- FIG. 27A: is an enlarged partial perspective view of the assembled syringe containing the full-face plunger ring, the plunger rod and the plunger rod tip shown in FIG. 27;
- FIG. 28: is a longitudinal cross-section of the plunger rod removed from the syringe taken along the line 28-28 of FIG. 27;
- FIG. 28A: is an enlargement of the cross-sectional view of the area indicated in FIG. 28;
- FIG. 28B: is a cross-sectional view of a plunger rod showing female threads thereon;
- FIG. 29: is a top plan view of the plunger rod shown in FIG. 28;
- FIG. 30: is a perspective view of the hill-face plunger ring shown in FIG. 27;
- FIG. 31: is a cross-sectional view of the full-face plunger ring shown in FIG. 30, the full-face plunger ring being positioned on a plunger rod tip, taken along the line 31-31 of FIG. 30.
- FIG. 32: is an enlarged partial cross-sectional view of the full-face plunger ring without the plunger rod tip in the barrel of a syringe;
- FIG. 33: is a longitudinal cross-section of another full-face plunger ring positioned on a plunger rod tip;
- FIG. 33A: is a top plan view of the full-face plunger ring shown in FIG. 33;
- FIG. 34: is a longitudinal cross-section of still another embodiment of the full-face plunger ring positioned on a plunger rod tip;
- FIG. 34A: is a top plan view of the full face plunger ring shown in FIG. 34;
- FIG. 35: is a longitudinal cross-section of a further embodiment of the full-face plunger ring positioned on a plunger rod tip;
- FIG. 35A: is a top plan view of the full-face plunger ring shown in FIG. 35;
- FIG. 36: is a cross-section of a further embodiment of full-face plunger ring designed for use with a plunger rod having thread means thereon;
- FIG. 36A: is an enlargement of thread means on the inside wall of the full-face plunger ring shown in FIG. 36;
- FIG. 36B: is a top plan view of the full-face plunger ring shown in FIG. 36;
- FIG. 37: is a cross-section of a still further embodiment of the full-face plunger ring having a plunger rod tip therein and is of a flat/horizontal top portion which interfaces with the content of the syringes; and
- FIG. 37A: is a top plan view of the full-face plunger ring shown in FIG. 37.

### DETAILED DESCRIPTION OF THE INVENTION

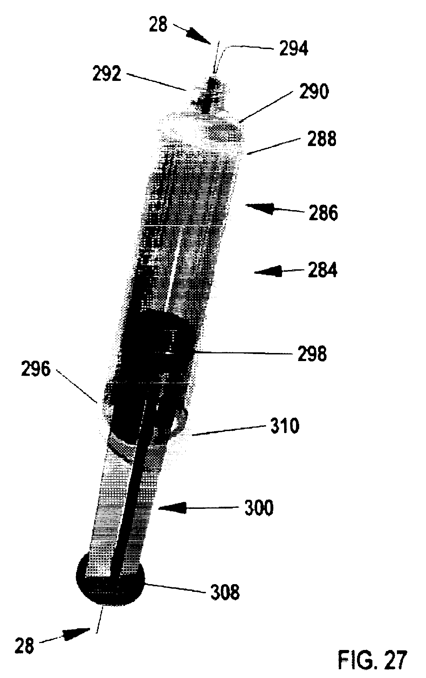

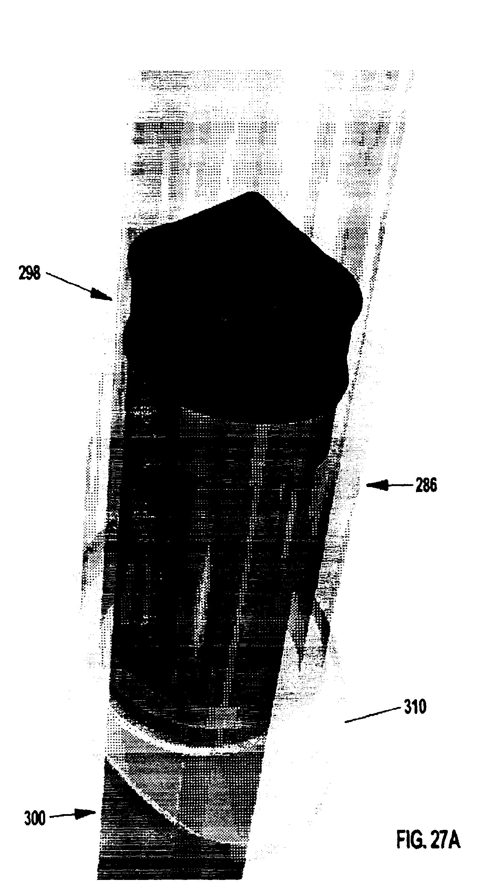

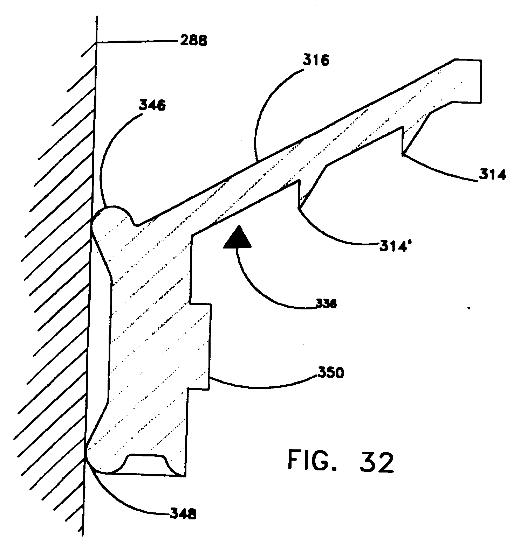

The present invention is directed to an elastomeric full-face plunger ring according to Figures 27 to 37A and to syringes and cartridges comprising said elastomeric full-face plunger ring. The syringes and cartridges here described are used to deliver fluids into a site, such as liquid pharmaceutical compositions and diagnostic contrast media delivered into a mammalian patient subcutaneously, intramuscularly or intravenously depending upon the particular medication to be administered. The barrel of the syringe is made of glass or polymeric materials and is equipped with a needle or luer connector having a tubing conduit attached thereto at the distal, tapered end thereof through which the fluids are delivered into the desired site. The syringes or cartridges may be pre-filled and sterilized ready for use, or they may be filled from a container, such as a vial, just prior to use. They may be used manually or in connection with power injectors well-known in the art.

The syringes or cartridges of the present invention may also be used to withdraw biological fluids from a patient, such as blood or tissue, for testing or other medical purposes.

The design of the thermoplastic plunger rod tip and the elastomeric plunger ring assembly used in the syringe or cartridge results in the following desirable characteristics:
reduction of breakaway forces;
reduction of running forces;
insuring against leakage;
reduction of contact area between the medical fluid and the rubber plunger; and
the elimination of siliconization.

The invention will now be described in reference to syringes, however, it is to be noted that the invention encompasses cartridges as well. Reference is now made to the drawings in describing the syringes in general and the thermoplastic plunger rod tip/elastomeric plunger ring assembly in particular detail which together constitute the invention.

Figures 27 to 37A refer to particular embodiments of the present invention.

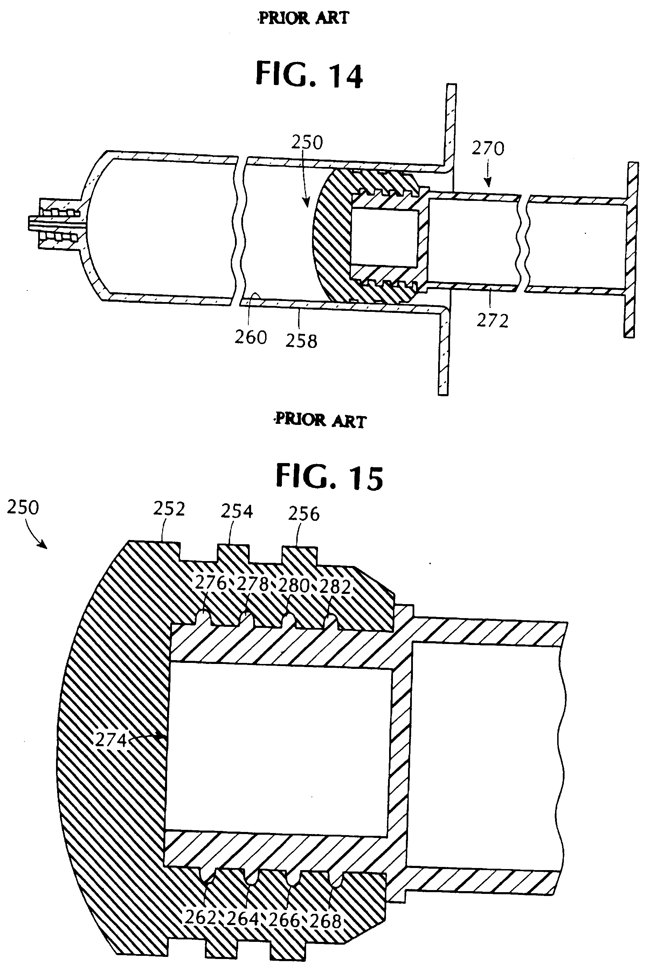

Figures 14 and 15 refer to embodiments of the state of the art used as comparison.

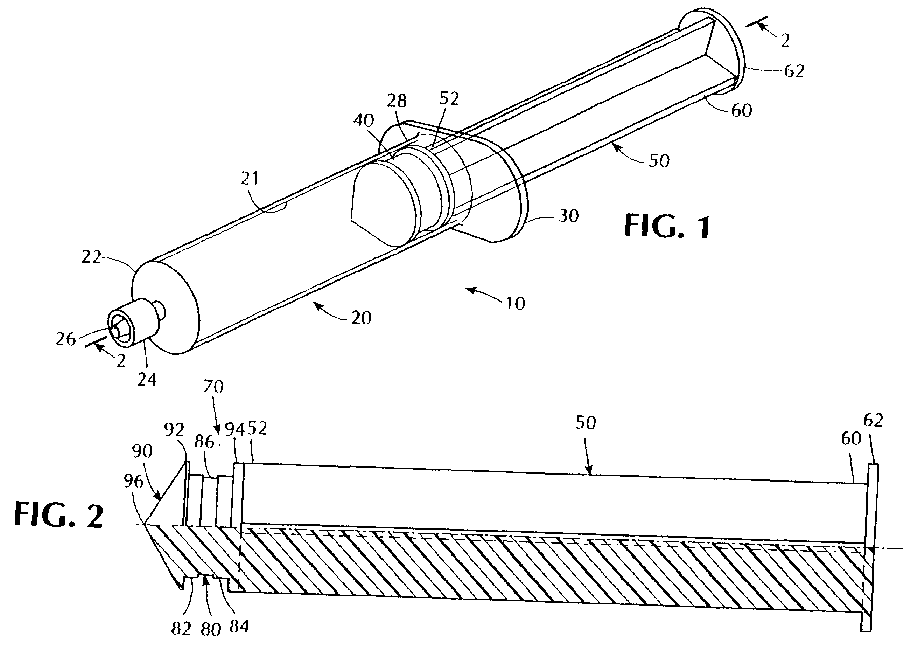

Figures 1 to 13 refer to other embodiments having parts in common with the embodiment of the present invention.

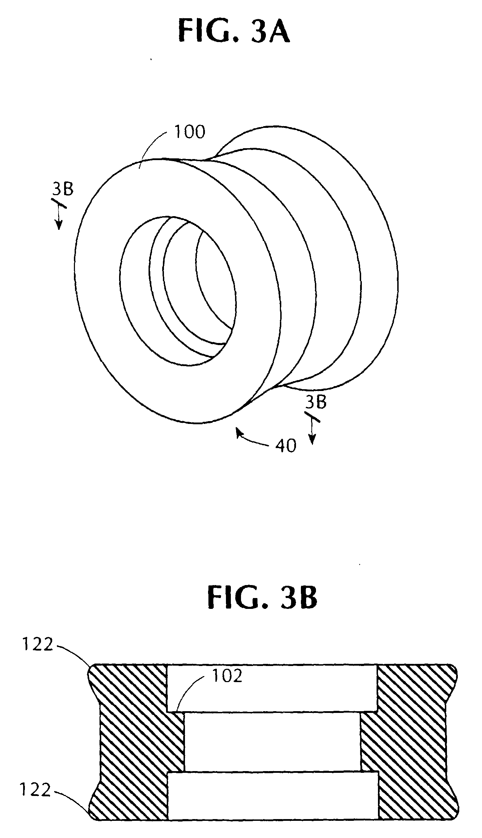

Referring to FIGS. 1 and 2, there is shown a syringe generally designated 10 comprising:
a barrel 20 having inside wall 21, a distal end 22 terminating in a tapered tip 24 which has bore 26 therethrough and proximal end 28 to receive plunger ring 40 to which plunger rod 50 is either removably attached or it may be integral with plunger 40. Plunger rod 50, having distal end 52 and proximal end 60 comprises handle 62 to facilitate exertion of force on plunger ring 40 by plunger rod 50 during operation of the syringe. Disposed about the periphery of proximal end 28 of barrel 20 is finger hub 30 which facilitates holding of barrel 20 during movement of plunger ring 40 by exerting a force on plunger rod 50 in order to move plunger ring 40 in a direction towards the distal end 22 of barrel 20 or in a direction toward the proximal end 28 of barrel 20.

Syringe barrel 20 is made of an inert gas impermeable, substantially transparent material, such as polyethylene, polypropylene, polystyrene, acrylic and methacrylic polymers and preferably of glass.

Plunger ring 40 is made of compressible, elastomeric materials including:
natural rubber;
acrylate-butadiene rubber;
cis-polybutadiene;
chlorobutyl rubber;
chlorinated polyethylene elastomers;
polyalkylene oxide polymers;
ethylene vinyl acetate;
fluorosilicone rubbers;
hexafluoropropylene-vinylidene fluoride-tetrafluoroethylene terpolymers, such as sold under the tradenames of Fluorel and Viton;
butyl rubbers;
polyisobutene, such as sold under the tradename Vistanex;
synthetic polyisoprene rubber;
silicone rubbers;
styrene-butadiene rubbers;
tetrafluoroethylene propylene copolymers; and
thermoplastic-copolyesters.
The durometer of the various elastomeric materials should preferably be in the range of from about 25 to about 80 Shore A.

Referring to FIG. 1, plunger ring 40 attached to plunger rod 50 is slideably received in barrel 20 by manual force exerted on plunge rod 50. Plunger ring 40 forms a sealing but slideable engagement with inside wall 21 of barrel 20.

Referring to FIG. 2, plunger rod 50, having proximal end 60 and distal end 52, further comprises plunger rod tip generally designated at 70. Plunge rod tip 70 having a mushroom shape configuration comprises: a cylindrical shaft 80, the distal end of which is designated at 82 and proximal end is designated at 84, and between the distal end 82 and proximal end 84 of cylindrical shaft 80 there is provided cylindrical recess or groove 86; and cone-shaped head 90 having a circular flange 92, zenith 96 and side 94 connecting flange 92 and zenith 96. Cone-shaped head 90 inside of barrel 20 moves freely without rubbing against inside wall 21 of barrel 20. It is intended to provide a surface area facing the content of the syringe barrel so that, because of its inert thermoplastic composition, no substantial interaction will occur between the content of the barrel and the plunger rod tip 70. Adjacent to proximal end 84 of cylindrical shaft 80, cylindrical shoulder 94 complete the embodiment of plunger rod tip 70. The outside diameter of cylindrical shoulder 94 at least approximates the outside diameter of flange 92 and, preferably is of the same diameter.

Plunger rod tip 70 can be integral with plunger rod 50 as shown in FIG. 2 or it can be removably attached to plunger rod 50 at the distal end 52 thereof.

When plunger rod tip 70 is intended for use in pre-filled syringes or cartridges, it is not permanently attached, that is, it is not integral with plunger rod 50. In this embodiment plunger rod tip is provided with female internal threads in the center portion thereof so that a male threaded plunger rod could be attached thereto.

Cylindrical shaft 80 having distal end 82, proximal end 84 and recess 86 thereon is designed to receive and firmly retain elastomeric plunger ring 40.

Referring to FIGS. 3A and 3B, plunger ring 40 is of cylindrical shape and is made of elastomeric materials. It comprises an inside wall 100, and an outside wall 120. Inside wall 100 comprises internal ring 102, slightly protruding out from inside wall 100 toward the center of the ring, designed to engage cylindrical recess or groove 86 on cylindrical shaft 80 thereby to form an internal seal between plunger ring 40 and plunger rod tip 70. Internal ring 102 is firmly held by recess or groove 86 so that upon movement of the plunger rod tip 40 in the barrel 20 of the syringe no disengagement can occur between plunger ring 40 and plunger rod tip 70. In all the embodiments of the present invention there must be at least one internal seal formed between said plunger ring and said plunger rod tip 70. However, more than one such seal may be provided in some of the embodiments depending on the intended use of the syringe.

Outside wall 120 of plunger ring 40 comprises at least one, but preferably more, plunger ring rib 122 designed to form secondary seal between it and the inside wall 21 of barrel 20. The configuration of rib or ribs 122 may be horizontal to a plane, or oriented towards the distal or proximal ends of the plunger rod tip 70.

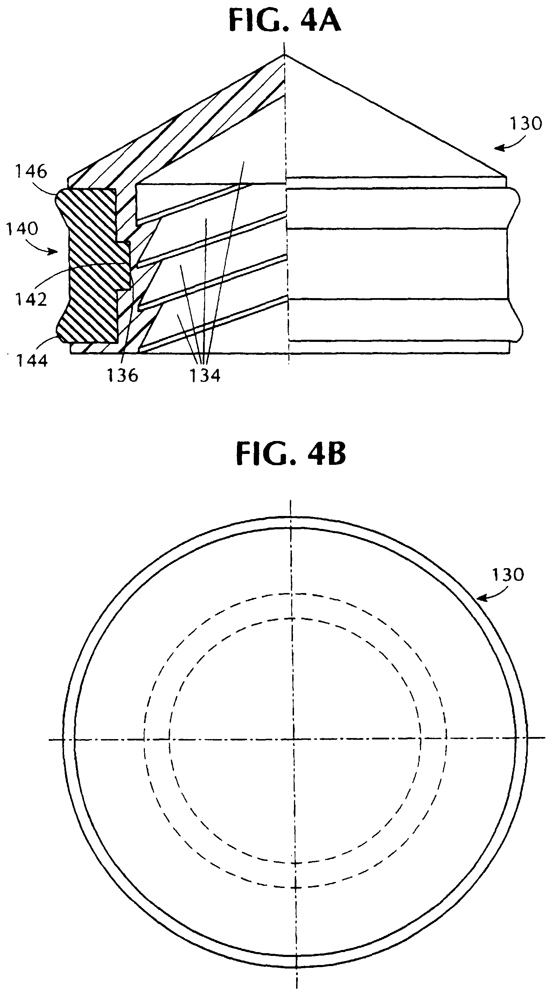

Referring to FIGS. 4A and 4B, there is shown in longitudinal cross-section and top plan view a plunger rod tip 130 with plunger ring 140 thereon, wherein the plunger rod tip 130 has internal female threads 134 therein for attachment to a male-threaded plunger rod.

Plunger ring 140 comprises: internal ring 142 forming an internal seal between it and recess or groove 136 in plunger rod tip 130; and plunger ring ribs 144 and 146 forming secondary seals with the inside wall of a barrel. As shown, two secondary seals are provided by the presence of the horizontal ribs insuring against leakage when the plunger rod tip is moved in the barrel in the proximal or distal direction.

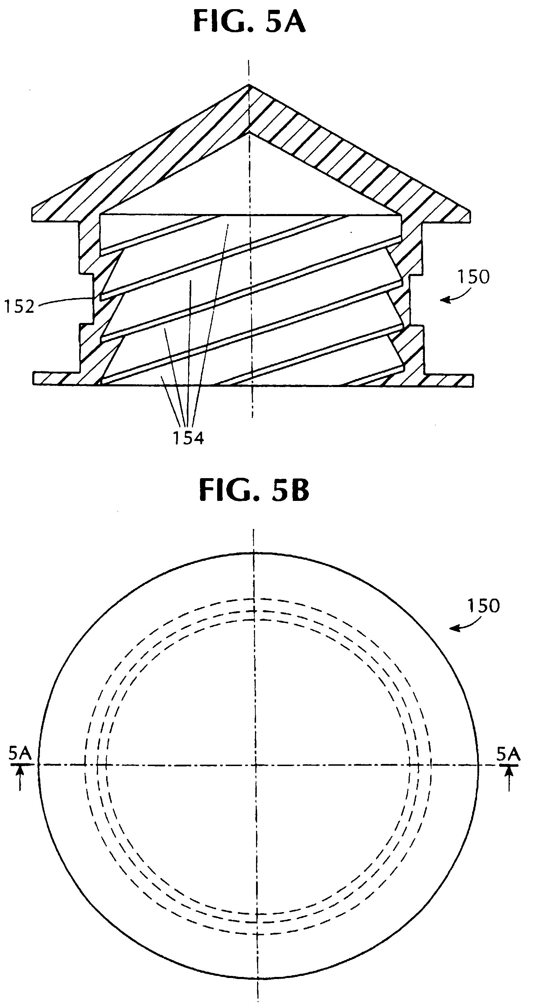

FIGS. 5A and 5B show the longitudinal cross-section and top plan view of a plunger rod tip 150 designed to carry one internal seal plunger ring thereon in recess or groove 152 wherein the plunger rod tip 150 has internal female threads 154 therein for attachment to a male-threaded plunger rod.

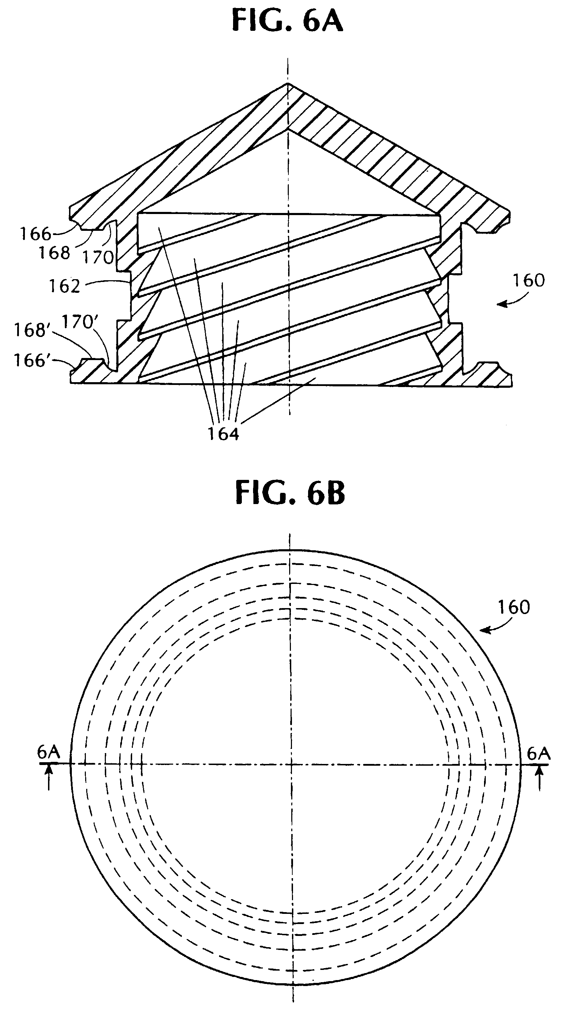

FIGS. 6A and 6B show another longitudinal cross section and top plan view of a plunger rod tip 160 designed to carry one internal seal plunger ring thereon in recess or groove 162 wherein the plunger rod tip 160 has internal female threads 164 thereon for attachment to a male-threaded plunger rod. The plunger rod tip 160 further comprises accentuated contours at 166, 168, 170, 166', 168' and 170' for insuring retention of a matching plunger ring on said plunger rod tip 160.

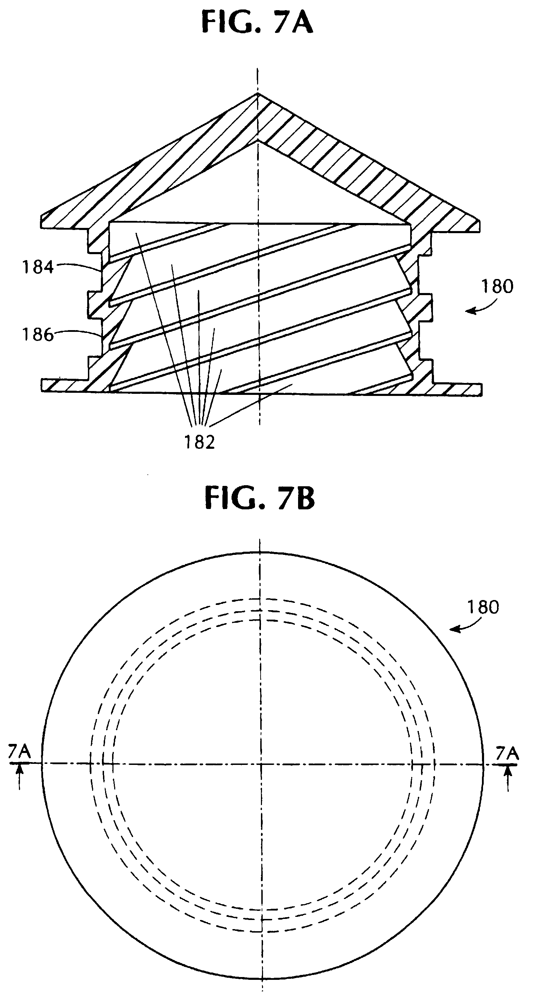

FIGS. 7a and 7B show another longitudinal cross section and top plan view of a plunger rod tip 180 designed to carry two internal seals plunger ring thereon in recess or groove 184 and 186, wherein the plunger rod tip 180 has internal female threads therein for attachment to a male-threaded plunger rod. The presence of two internal seals further insures against leakage at the interface between the plunger rod tip and the plunger ring.

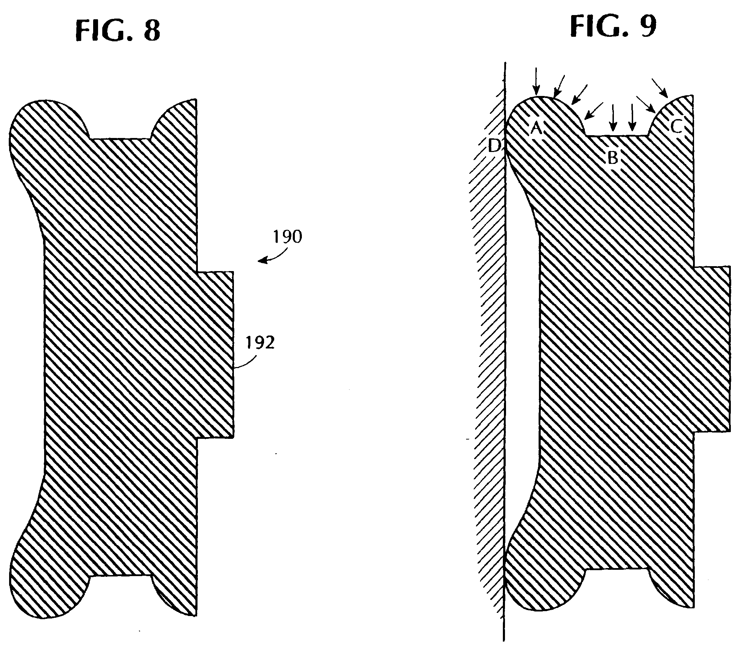

FIG. 8 shows a longitudinal cross-section of a plunger ring 190 having one internal seal provision at 192 with a matching plunger rod tip such as shown in FIGS. 6A and 6B.

FIG. 9 shows the longitudinal cross-section of the plunger ring 190 having one internal seal shown in FIG. 8 and the directions of pressures exerted on the plunger ring in use.

The plunger ring has minimal contact areas with the inside wall of the barrel. The various designs described in the embodiments of the present invention use a hydraulic action to increase the contact area as the pressure increases during use. In FIG. 9, as illustrated, as pressure increases: area A is forced toward the side of the barrel D; area C is forced towards the plunger tip; and area B is compressed. The same hydraulic action works in both directions, i.e. when the plunger rod tip is forced to travel in the distal or proximal directions.

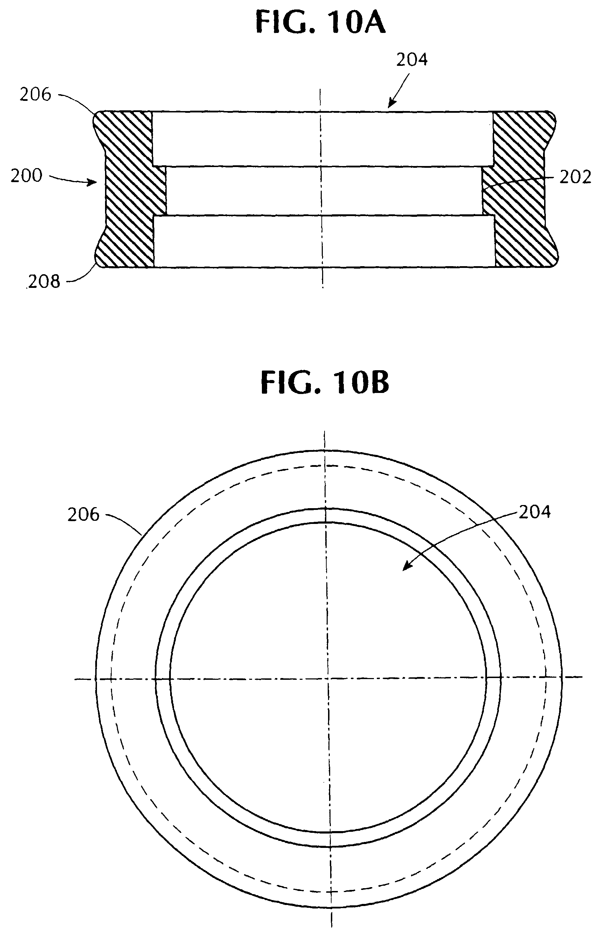

FIGS. 10A and 10B show the longitudinal cross-section and side elevational view of still another plunger ring 200 having one internal seal 202 in place on the plunger rod tip 204 wherein the plunger ring comprises plunger ring rims 206 and 208.

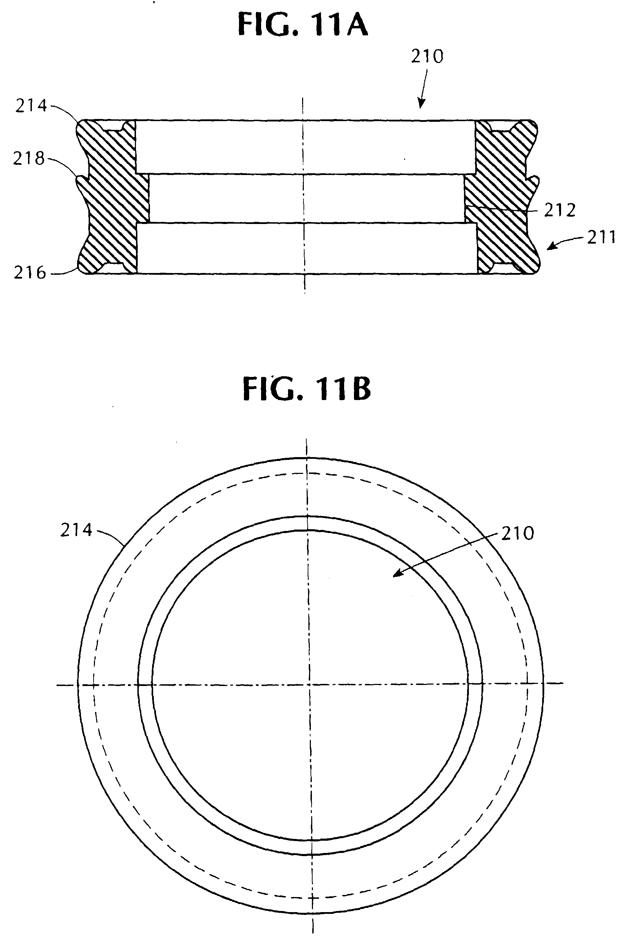

FIGS. 11A and 11B show the longitudinal cross section of still another plunger rod tip 210/plunger ring 211 combination having one internal seal 212 in place on the plunger rod tip, wherein the plunger ring comprises plunger ring rims 214, 216 and 218. These rims are designed to provide leak-proof protection under pressure in one direction. As shown in the drawings leak-proof protection is achieved when the plunger ring/plunger tip combination is forced to travel distally, such as when the syringe is used for injection. If the syringe is intended for use in withdrawing biological fluid from a patient, the orientation of the plunger ring rib/plunger rod tip combination would be reversed 180°.

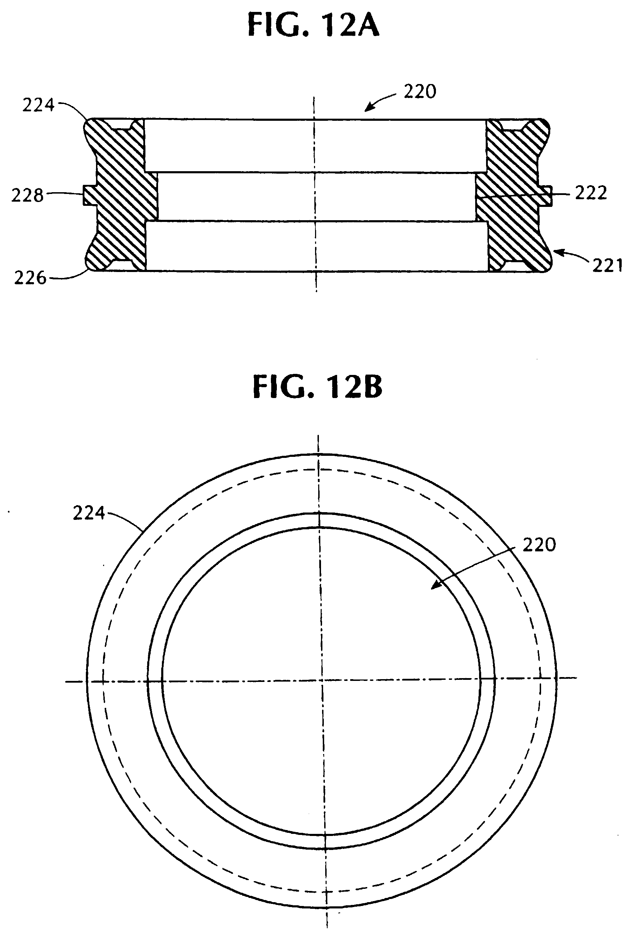

FIGS. 12A and 12B show a longitudinal cross-section and side elevational view of still another plunger rod tip 220/plunger ring 221 combination having an internal seal 222 in place on the plunger rod tip, wherein the plunger ring comprises plunger ring rims 224, 226 and 228. This embodiment of the present invention is designed to provide leak-proof protection in both the proximal and distal directions.

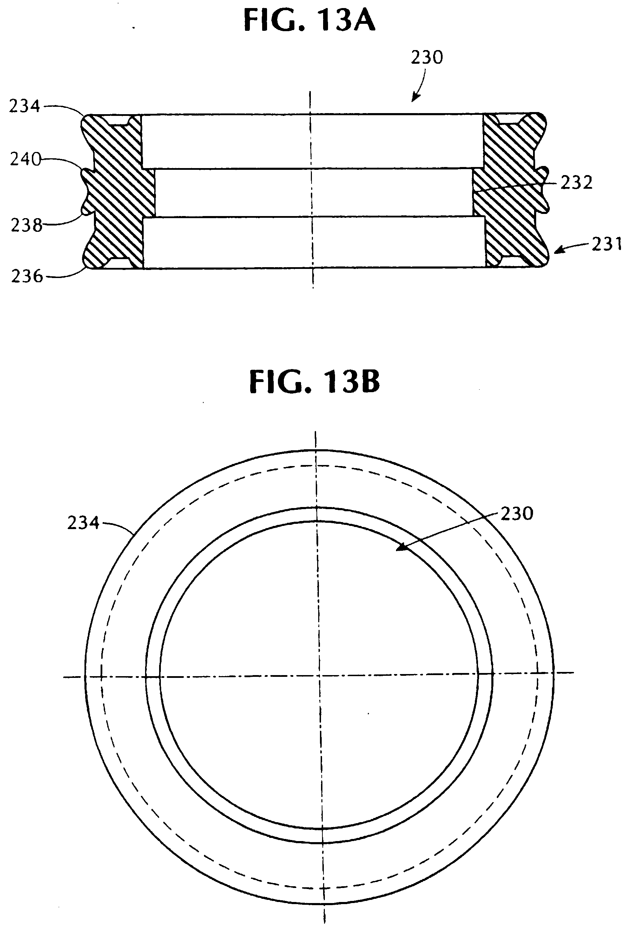

FIGS. 13A and 13B show a longitudinal cross-section and side elevational view of still another plunger rod tip 230 having an internal seal 232 in place on the plunger rod tip/plunger ring 231 combination, wherein the plunger ring comprises plunger ring rims 234, 236 and 238 and 240. This embodiment of the present invention is also designed to provide leak-proof protection in both the proximal and distal directions.

FIGS. 14 shows a longitudinal fragmentary cross-section of a typical prior art syringe, plunger and plunger rod; and FIG. 15 show an enlarged fragmentary cross-section thereof. Plunger 250, made of elastomeric material, comprises external plunger rims 252, 254 and 256 which are designed to provide a leak-proof seal between the plunger and the inside wall 260 of barrel 258. Plunger 250 is provided with female threads 262, 264, 266 and 268 to receive a male-threaded plunger rod. Plunger rod 270 of this prior art embodiment comprises shank 272 and plunger rod head 274 which is provided with male threads 276, 278, 280 and 282 to engage female threads 262, 264, 266 and 268. It is apparent that the content of the barrel, such as a medical solution, interfaces with the elastomeric plunger thereby allowing undesirable interaction therebetween. Such interaction may result in the dissolution of at least small amounts of chemicals present in the elastomeric material which then taints the medical fluids.

An embodiment of the present invention shown in FIG. 1 was comparatively tested against a corresponding prior art device illustrated in cross-section in FIGS. 14 and 15. The summary of the test results expressed as averages in lbs is shown in Table I.

**TABLE I**

| **20 ml Syringe Test Data** | | |
|---|---|---|
| | **Present Invention** | **Prior Art** |
| Breakaway Force With Silicone | 0.15 lbs | 4.3 lbs |
| Breakaway Force Without Silicone | 0.61 lbs | 16.9 lbs |
| Running Force With Silicone | 0.17 lbs | 2.5 lbs |
| Running Force Without Silicone | 0.60 lbs | 11.6 lbs |
| Leakage | None | Some observed between plunger ribs |
| Test to Failer (Breakage) | 90.0 lbs | 86.0 lbs |

In addition to the extremely low breakaway and running forces shown in Table I, contact between the medical fluid in the syringe barrel and the elastomeric (rubber) material constituting a portion of the plunger of the present invention was reduced about 80%, thereby reducing the tendency of the presence of elastomeric exudate in the medical fluid.

Description of the details of the comparative testing follows.

Testing was conducted using three pre-filled syringes for each test. Measurements were made with a standard Test Gauge at five points in the barrel of the syringes as the plunger was forced to travel down towards the distal end of the syringe:
Test Point 1 was at the proximal end of the barrel;
Test Point 2 was at about 25% down from the proximal end of the barrel;
Test Point 3 was at about 50% down from the proximal end of the barrel;
Test Point 4 was at about 75% down from the proximal end of the barrel; and
Test point 5 was at the distal end of the barrel, close to where the barrel tapers to form the tip.

The result of the testing of the syringes are shown in the following Figures.

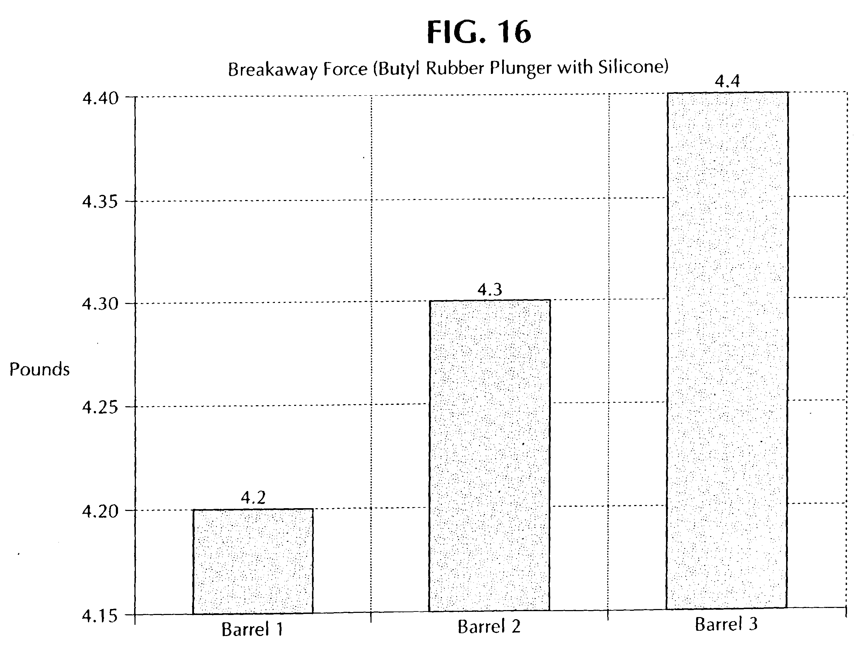

FIG. 16 shows a graph of breakaway forces between the inside walls of three barrels and butyl rubber plungers with silicone of the prior art, wherein Barrel 1, Barrel 2 and Barrel 3 show a breakaway force of 4.2, 4.3 and 4.4 pounds, respectively.

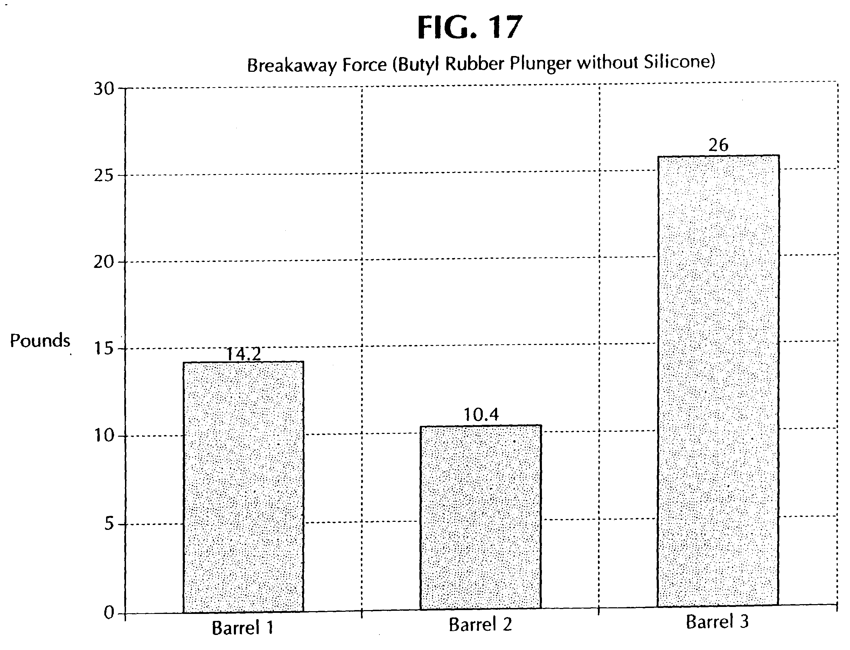

FIG. 17 shows a graph of breakaway forces between the inside walls of three barrels and butyl rubber plungers without silicone of the prior art, wherein Barrel 1, Barrel 2 and Barrel 3 show a breakaway force of 14.2, 10.4 and 26 pounds, respectively. It is apparent that without the use of silicone the breakaway forces have substantially increased.

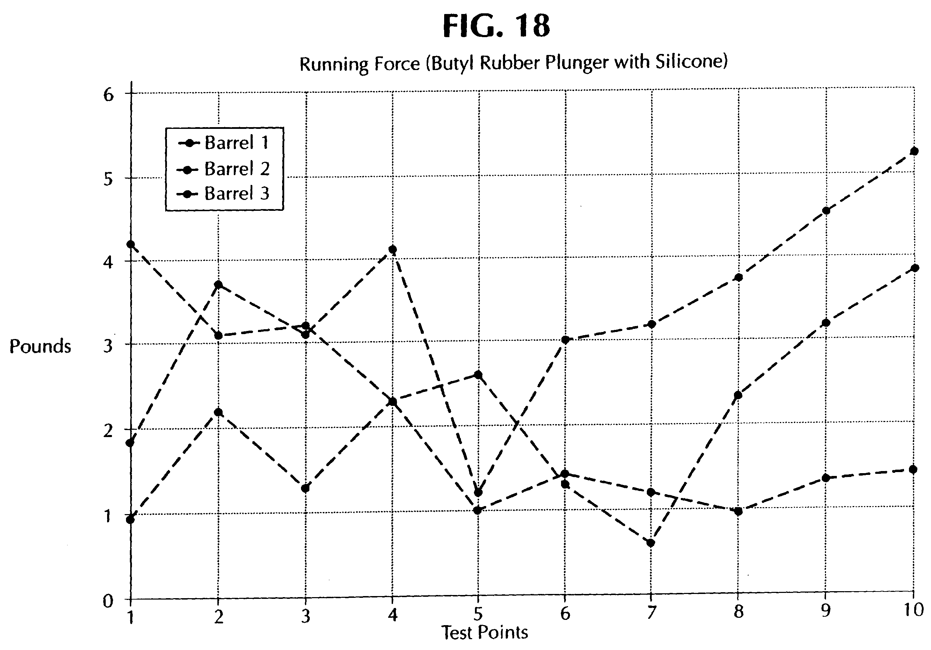

FIG. 18 shows a graph of running forces between the inside walls of three barrels and butyl rubber plungers with silicone of the prior art, wherein the running forces are shown to vary from about 0.75 pounds to about 5.2 pounds.

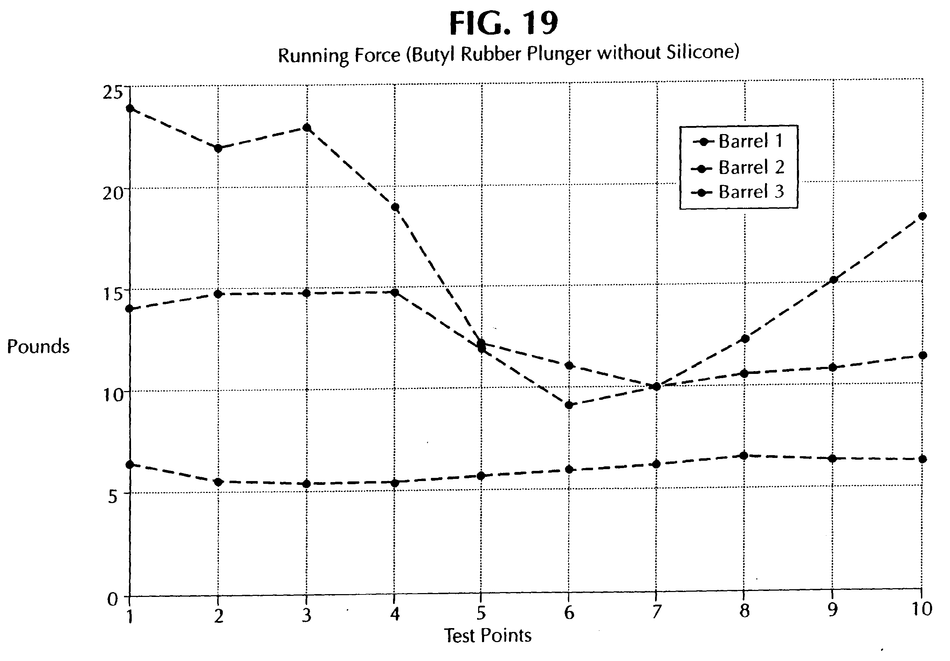

FIG. 19 shows a graph of running forces between the inside walls of three barrels and butyl rubber plungers without silicone of the prior art, wherein the running forces are shown to vary from about 12 pounds to about 24 pounds. Again, it is apparent that without the use of silicone the running forces have substantially increased, similarly to the breakaway forces shown in FIGS. 16 and 17.

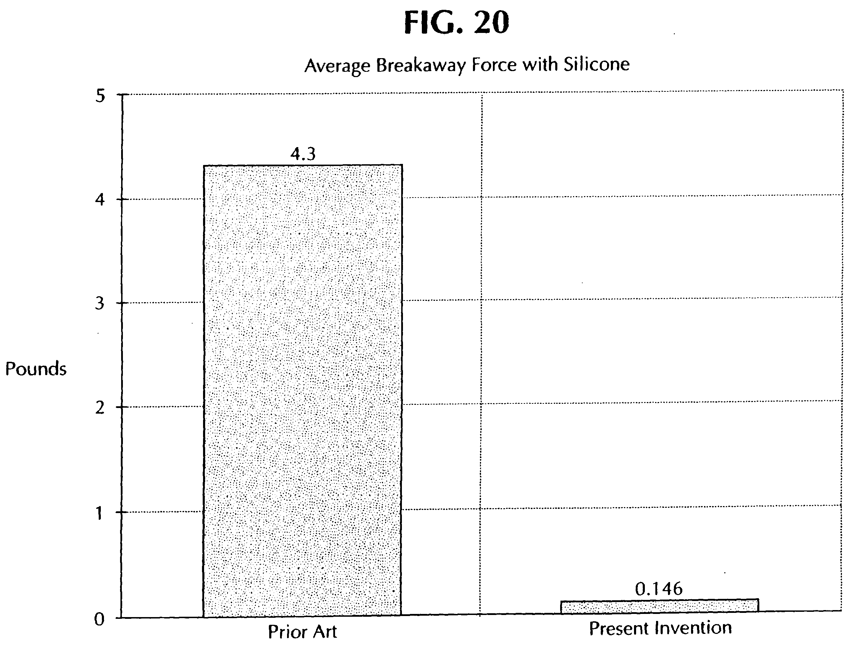

FIG. 20 shows a graph of average breakaway forces between the inside walls of the barrels and the butyl rubber plungers with silicone of the prior art, and the average breakaway forces between the inside walls of the barrels and the plunger rod tip/plunger ring combination with silicone of the present invention.

It is apparent from the graphs that the average breakaway force of the prior art device is many-fold larger than that of the present invention.

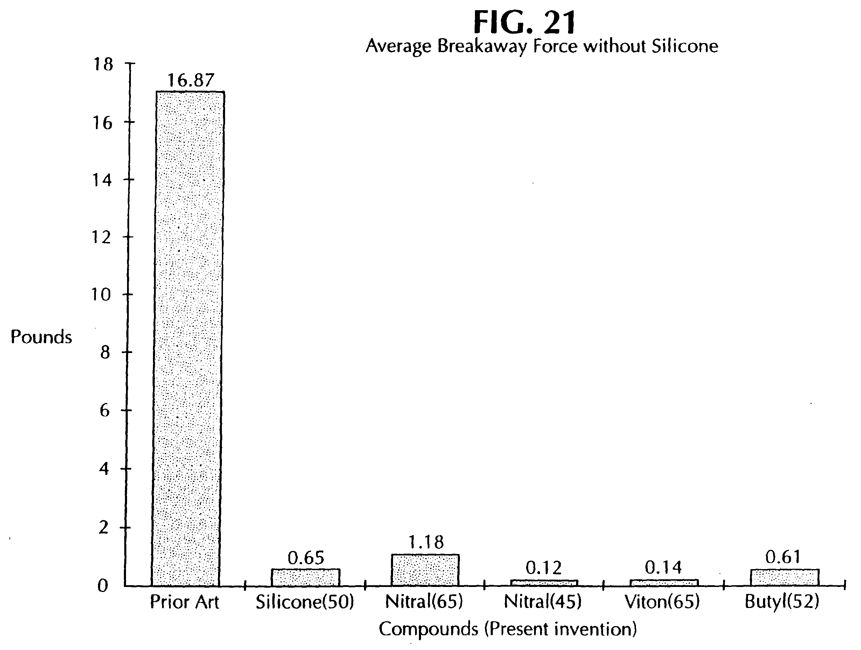

FIG. 21 shows a graph of average breakaway forces between the inside walls of the barrels and the butyl rubber plungers without silicone of the prior art, and the average breakaway forces between the inside walls of the barrels and the plunger rod tip/plunger ring combination without silicone of the present invention wherein the plunger ring is made of Silicone(50), Nitrial(65), Nitrial(45), Viton(65) or Butyl(52) rubbers the average breakaway force for each of which is shown in the graph.

The breakaway forces of the prior art device is at least 25, 14, 140, 120 and 27 times larger than the breakaway forces of the present invention respectively, using the rubber materials from which the plunger rings are made.

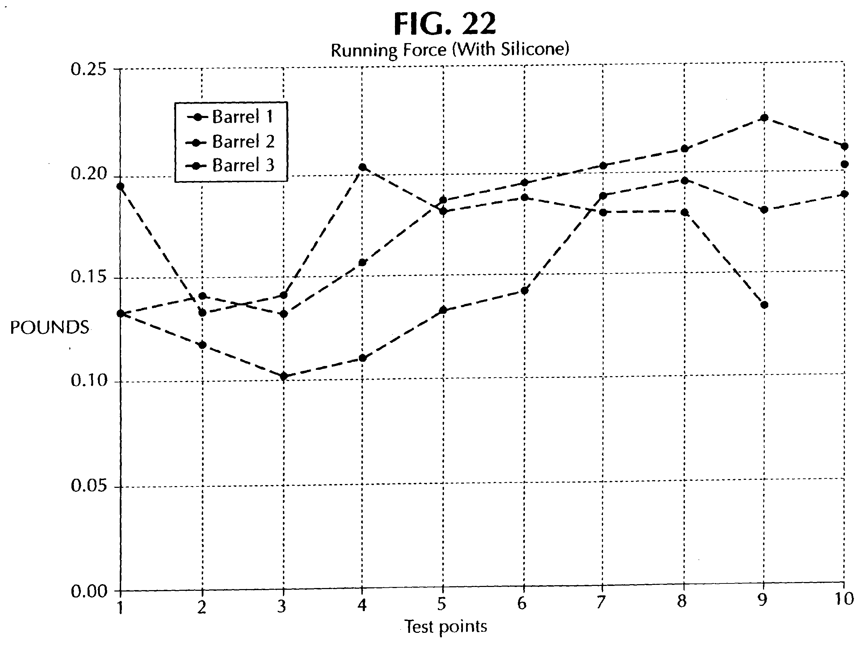

FIG. 22 shows a graph of running forces between the inside wall of the barrel and the plunger rod tip/butyl rubber plunger ring combination with silicone of the present invention showing the result for each of three barrels.

The running forces for Barrel 1 range of from about 0.1 lbs to about 0.18 lbs, the running forces for Barrel 2 range of from about 0.13 lbs to about 0.21 lbs, and the running forces for Barrel 3 range of from about 0.13 lbs to about 0.23 lbs.

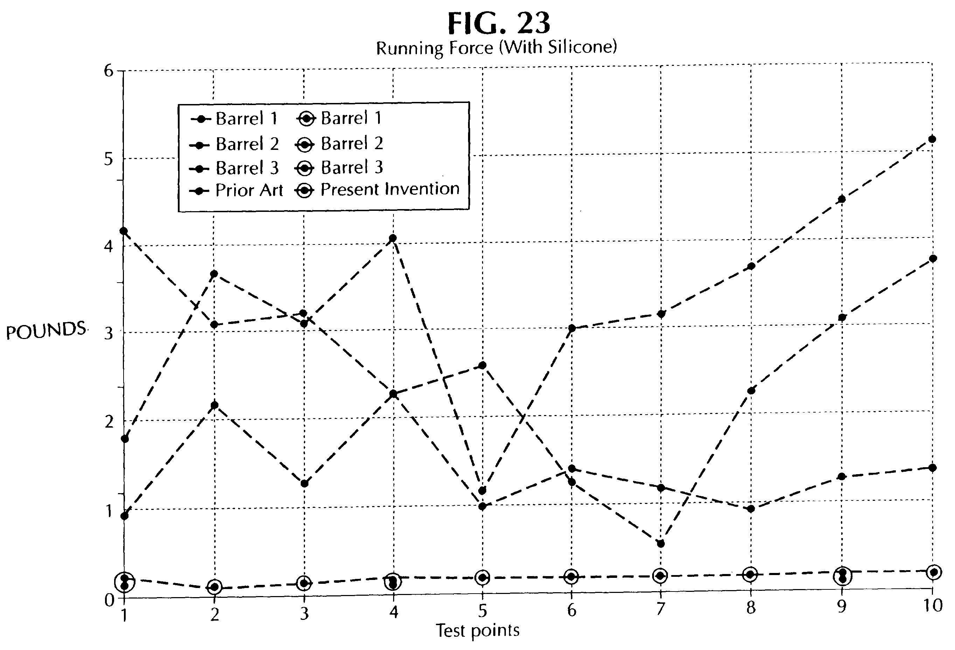

FIG. 23 shows a graph of running forces between the inside wall of the barrel and the plunger rod of the prior art made of butyl rubber with silicone, versus a graph a running forces between the inside wall of the barrel and the plunger rod tip/plunger ring combination made of butyl rubber with silicone of the present invention.

As shown, the running forces of the prior art device are in the range of from about 0.60 lbs to about 5.2 lbs, while the running forces of the present invention is in the range of about 0.25 lbs.

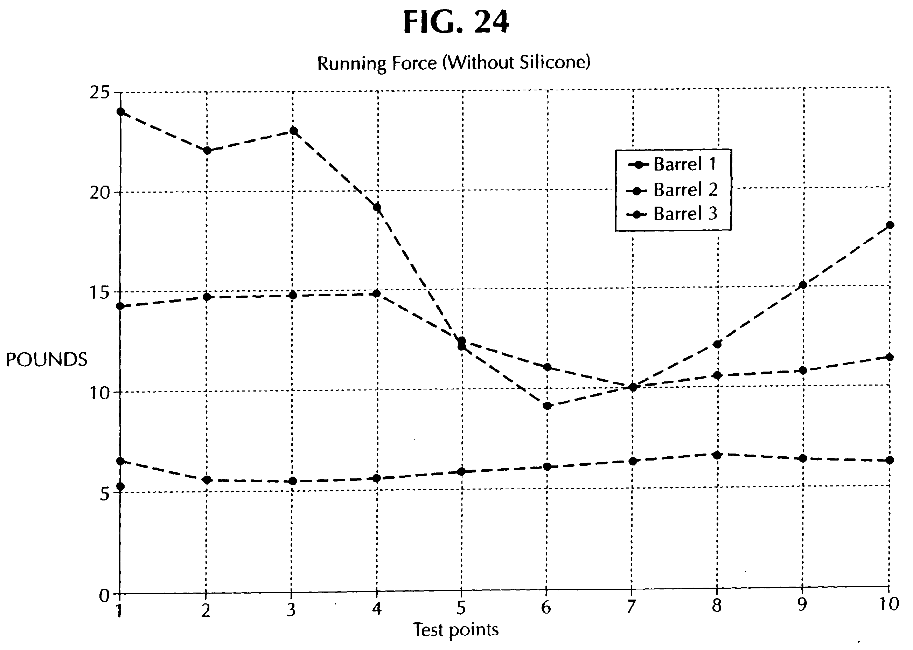

FIG. 24 shows a graph of running forces between the inside wall of the barrel and the plunger made of butyl rubber without silicone of the prior art for Barrels 1, 2 and 3.

For Barrel 1, the running forces range from about 5.5 lbs to about 6.0 lbs, for Barrel 2, the running forces range from about 7.0 lbs to about 12.0 lbs, and for Barrel 3 the running forces range from about 24.0 lbs to about 18.0 lbs.

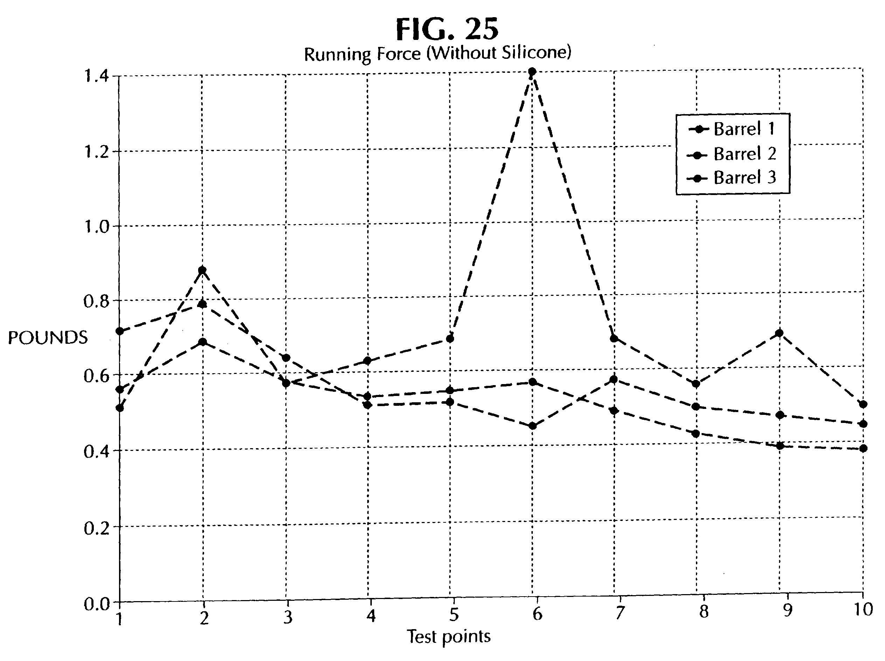

FIG. 25 shows a graph of running forces between the inside wall of the barrel and the plunger rod tip/plunger ring combination without silicone of the present invention for Barrels 1, 2 and 3.

For Barrel 1, the running forces range from about 0.50 lbs to about 1.40 lbs, for Barrel 2, the running forces range from about 0.40 lbs to about 0.65 lbs, and the running forces for Barrel 3 range from about 0.80 lbs to about 0.45 lbs.

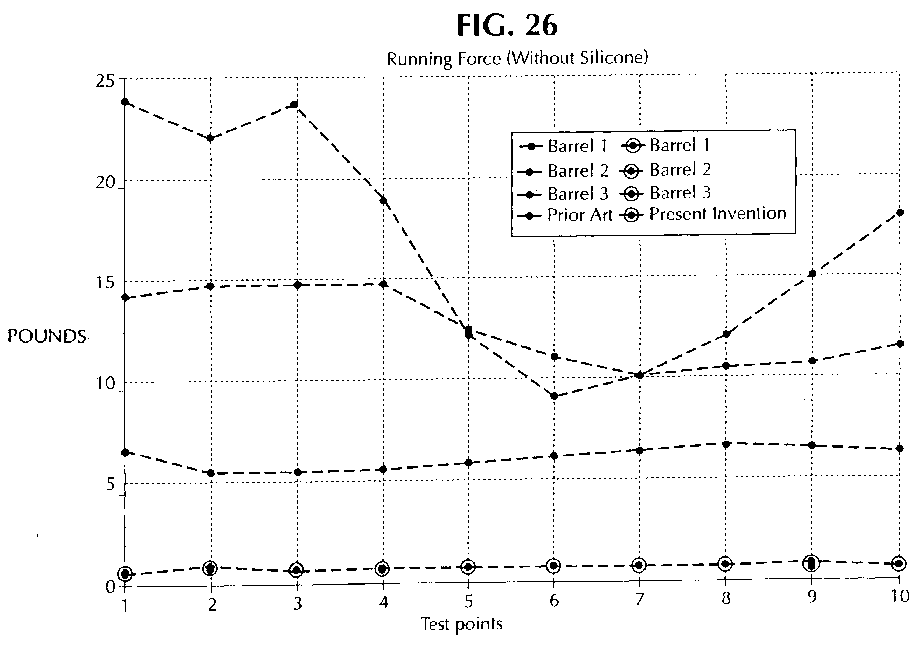

FIG. 26 shows a graph of running forces between the inside wall of the barrel and the plunger made of butyl rubber without silicone of the prior art for Barrels 1, 2 and 3, versus a graph of running forces between the inside wall of the barrel and the plunger rod tip/plunger ring combination made of butyl rubber without silicone of the present invention.

It is apparent from the graphs that the running forces existing in the prior art without the use of silicone are large, while the running forces existing in the present invention without silicone are very low.

For the prior art the running forces for Barrel 1 range of from about 5.5 lbs to about 6.0 lbs, for Barrel 2 the running forces range of from about 9.0 lbs to about 11.5 lbs, and for Barrel 3 the running forces range of from about 23.5 lbs to about 18.0 lbs.

In further embodiments of the present invention a full-face plunger ring is provided in conjunction with a plunger rod tip or with a plunger rod tip integral with a plunger rod. During the course of extensive experimentation applicants have discovered that low breakaway and running forces may be achieved with the full-face plunger ring similar to that obtained with a plunger ring where the plunger rod ring constitutes an interface of only 5 to 9% with the content of the syringe or cartridge, the remaining interface between the content and the thermoplastic plunger rod tip being about 95% to about 91%. These embodiments are intended for use with essentially inert fluids contained in the barrel of a syringe or cartridge, in order to avoid leaching of trace amounts of ingredients from the elastomeric plunger ring being in contact with the fluid content of the barrel of the syringe or cartridge. These embodiments are illustrated in FIGS. 27 to 37A the description of which follow.

Certain components of the present embodiments referred to in FIGS. 27 to 37A are the same as described in connection with FIGS. 1 to 15. Referring to FIGS. 27, 27A, 28A, 28B, 29, 30, 31, and 32, there is shown a syringe generally designated 284, a full-face plunger ring generally designated 298, a plunger rod generally designated 300 and a plunger rod tip generally designated as 302. syringe 284 comprises:
a barrel 286 having inside wall 288,
a distal end 290 terminating in a tapered tip 292 which has a bore 294 therethrough, and
proximal end 296 to receive full-face plunger ring 298.

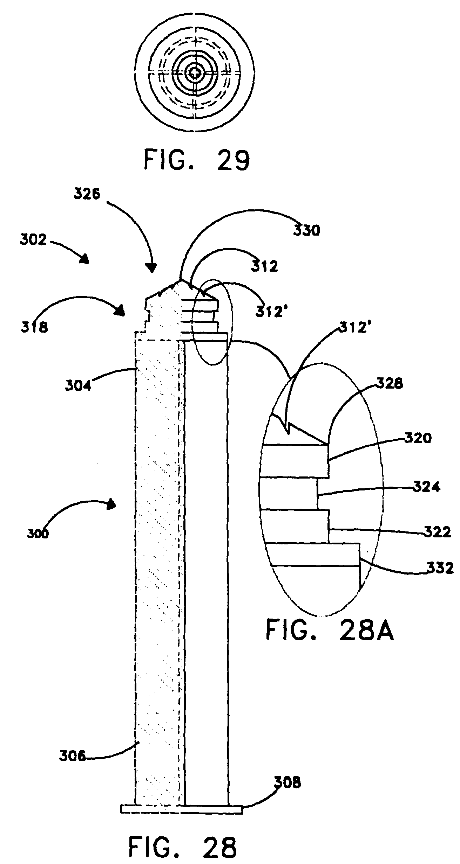

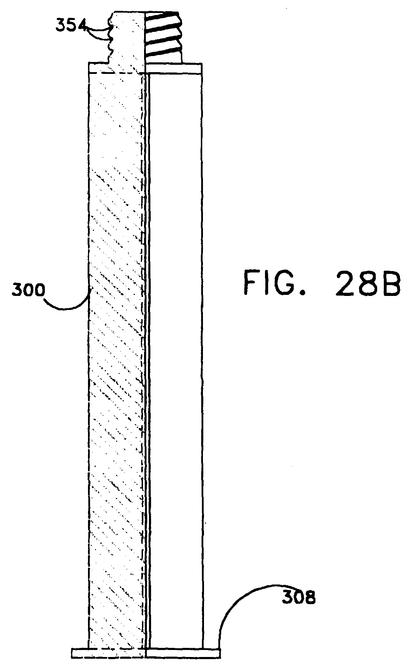

Full-face plunger ring is positioned over plunger rod tip 302 and interfaces the fluid content of the barrel 286. Plunger rod tip 302 is either removably attached to plunger rod 300 or is integral therewith. In prefilled cartridges or syringes, plunger rod tip 302 carrying full-face plunger ring 298 is positioned in the proximal end 296 of barrel 286 and seals the content of the barrel. Prior to administration of the content to a patient, plunger rod 300 is connected to plunger rod tip 302. Both the plunger rod tip and plunger rod are equipped with thread means. Plunger rod 300 and plunger rod tip 302 of the one piece configuration are shown in FIG. 28 wherein the plunger rod has a distal end 304 and proximal end 306. The proximal end is provided with handle 308, which is integral with the proximal end of the plunger rod and is designed to facilitate exertion of force on plunger rod during operation of the syringe. Disposed about the periphery of proximal end 296 of barrel 286 is finger hub 310 which facilitates holding of barrel 286 during movement of full-face plunger ring 298 in a direction toward the distal end 290 of barrel 286 or in a direction towards the proximal end 296 of barrel 286.

Fluid injection and withdrawal is accomplished manually. However, for certain injections it is preferred to use the syringe of the present invention in conjunction with a power injector. For example, in urography and angiography intravenous injection of a contrast medium is used to render the urinary tract, arteries or veins of a patient opaque. The coated vascular structures are then radiographically imaged for diagnostic purposes. For delivery to the desired site, the contrast medium is placed in the syringe and forced through the needle or catheter such that the contrast medium enters the blood stream or the bladder. As the contrast medium is being injected to the site to be visualized, high pressures are often encountered, sometimes as high as 1,000 psi. This requires a rather high force to be exerted on the plunger rod in order to deliver the contrast media from the syringe. In addition such force is to be delivered in a contrast manner for continuous and even volume delivery of the contrast medium.

The present invention may be used in such power delivery injectors in order to reduce reliance on an assistant enabling the operator to be in complete control of the injection of the contrast medium and to deliver precise volume at a constant rate. Power injectors of the hydraulic, pneumatic and electric types may be used with the present invention. Hydraulic injectors have an electric motor connected to a hydraulic pump which drives a ram connected to the syringe that contains the contrast medium. In pneumatic injectors the source of power is a compressed gas supplied from a tank or a compressor. Electric injectors are powered by electric motors in which a transmission means serves to change circular motion into linear motion which then drives a ram.

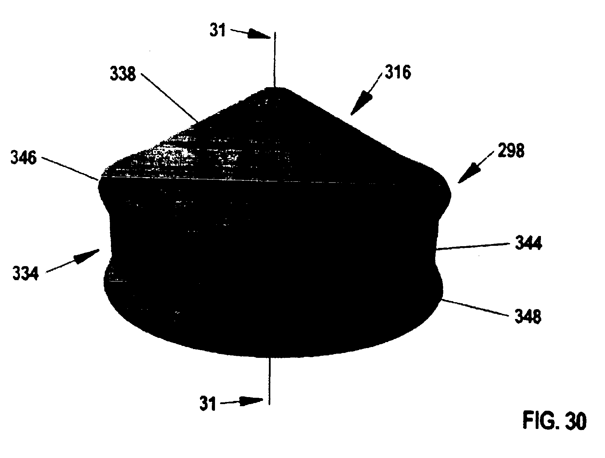

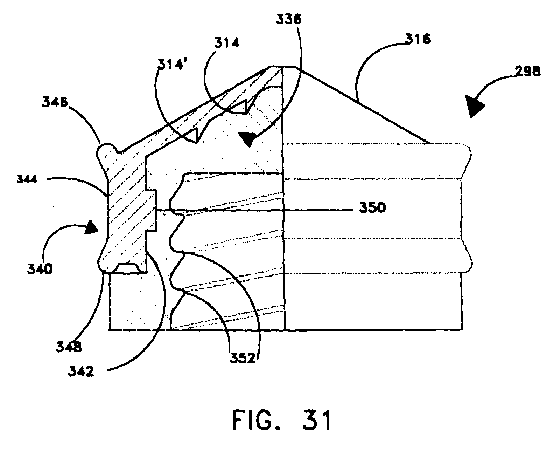

As shown in FIGS. 28 and 28A, plunger rod tip 302 comprises a cap portion, comprising a zenith 330 and a plurality of notches 312 and 312' while the full-face plunger ring 298 of FIGS. 30 and 31 is provided with a plurality of tooth-like projection 314 which engage the plurality of notches in the plunger rod tip for preventing the full-face plunger ring from moving/stretching toward the outside diameter of the full-face plunger ring and securely holding the top portion 316 of the full-face plunger ring 298 in place under pressure.

Referring again to FIGS. 28 and 28A, plunger rod tip 302 having a mushroom-shape configuration further comprises: a cylindrical shaft generally designated 318, the distal end of which is designated at 320 and the proximal end of which is designated 322, and between the distal end and proximal end of the cylindrical shaft there is provided a recess or groove 324; and a cone-shaped head generally designated as 326 having a circular flange 328, zenith 330 and cylindrical shoulder 332 connecting flange 328 and zenith 330. Adjacent to the proximal end 322 of cylindrical shaft 318, cylindrical shoulder 332 completes the embodiment of plunger rod tip 302. The outside diameter of cylindrical shoulder 332 is larger than the outside diameter of circular flange 328.

Recess or groove 324 in cylindrical shaft of plunger rod rip and notches 312 and 312' will engageably receive full-face plunger ring 298 which comprises:
a cone-shaped top portion 316 having an inside wall 336 and an outside wall 338; and a cylindrical portion 334. Cylindrical portion 334 comprises a right cylinder 340 having an inside wall 342 and an outside wall 344; a distal rim 346 protruding away from said outside wall 338 of right cylinder 340; and proximal rim 348 protruding away from said outside wall 344 of right cylinder 340. Inside wall of right cylinder 342 is provided with a circular band 350 protruding toward the center of plunger rod tip 302 which engages the recess or grooves 324 of plunger rod tip 302. When the full-face plunger ring is positioned on plunger rod tip, notches 312 and 312' will engage the tooth-like projections 314 and 314', recess or groove 324 in plunger rod tip will engage circular band 350 on the full-face plunger ring 298. This feature ensures that during operation of the syringe the two components will be securely held together.

Distal rim 346 during injection under pressure flexes toward the inside wall 288 of barrel 286 thereby providing an external seal between it and the inside wall of the barrel. During withdrawal of fluid from a source, proximal rim 348 is flexed toward the inside wall 288 of the barrel 286. The force exerted on the rims during injection or withdrawal of fluid presses the rims against the wall of the barrel to insure leak-proof containment of the fluid in either the distal or proximal travel of the full-face plunger ring.

The plunger rod tip contains male threads 352 for engagement with female threads 354 for engagement of plunger rod 300.

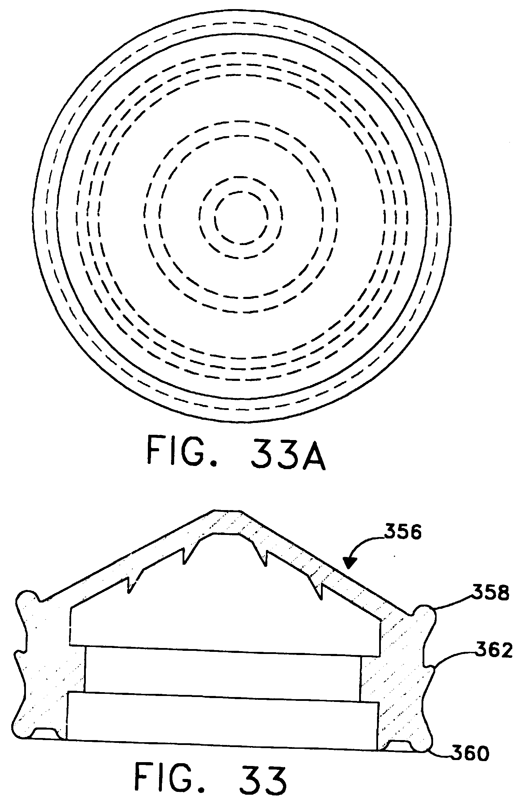

FIG. 33 is a longitudinal cross-section of another full-face plunger ring 356 positioned on a plunger rod tip, and FIG. 33A is a top plan view thereof. Full-face plunger ring comprises distal rim 358, proximal rim 360 and center rim 362. Distal and center rims are oriented in distal direction while proximal rim is oriented in the proximal direction. The configuration of the rims offers leak-proof protection when the device is operated distally, i.e., when the syringe is used for injection.

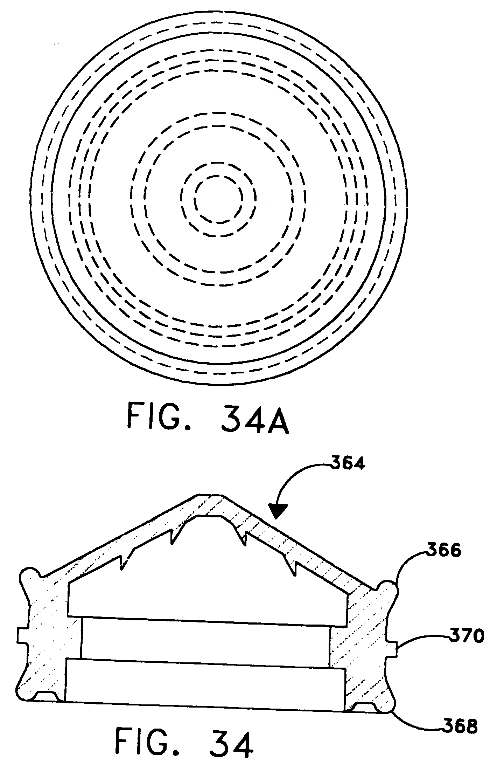

FIG. 34 shows a longitudinal cross-section of still another full-face plunger ring 364 positioned on a plunger rod tip, and FIG 34A shows a top plan view thereof The full-face plunger ring comprises distal rim 366 oriented in distal direction, proximal rim 368 oriented in proximal direction, and center rim 370 oriented horizontally towards the inside wall of a syringe or cartridge barrel. This embodiment of the present invention is designed to provide leak-proof protection in both the proximal and distal directions.

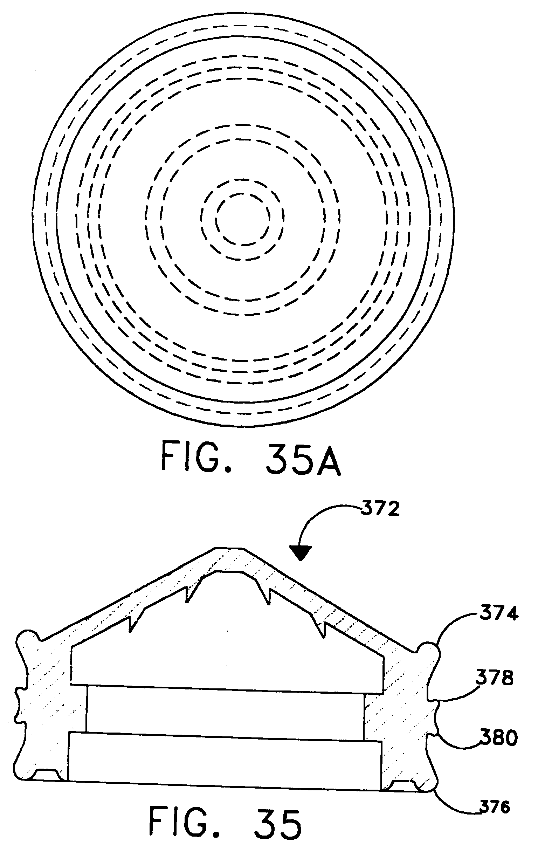

FIG. 35 shows a longitudinal cross-section of a further embodiment of the full-face plunger ring 372 positioned on a plunger rod tip, and FIG. 35A shows a top plan view thereof. The full-face plunger ring comprises: distal rim 374 oriented in distal direction; proximal rim 376 oriented in proximal direction; and two center rims 378 and 380, one oriented in the distal direction while the other is oriented in proximal direction. This embodiment of the present invention is also designed to provide leak-proof protection in both the proximal and distal directions.

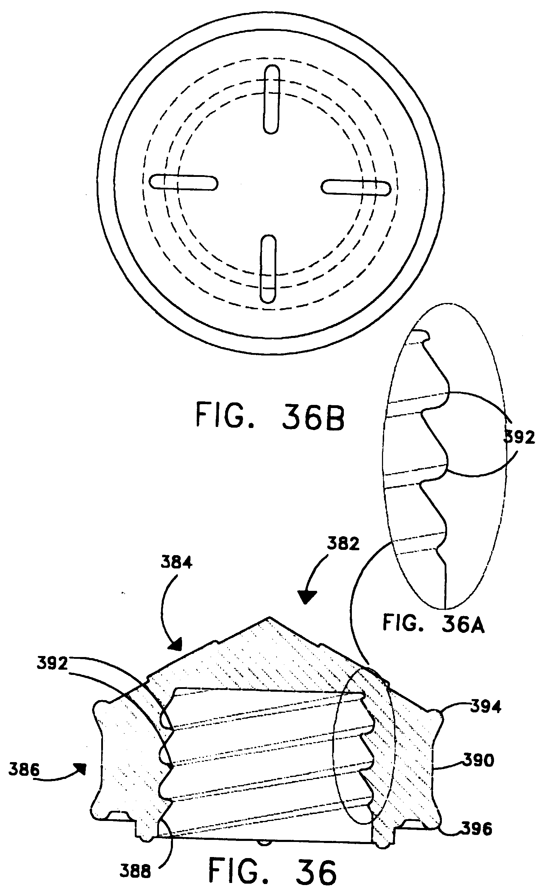

FIG. 36 shows still a further embodiment of the full-face plunger ring 382 without being supported by a plunger rod tip. This design is intended for use with the plunger rod 300 shown in FIG. 28B. FIG. 36 shows a cross-sectional view of the full-face plunger ring; FIG. 36B shows an enlargement of the cross-sectional view of the area indicated in FIG. 36; and FIG. 36B shows a top plan view of the full-face plunger ring. The full-face plunger ring comprises: a top conical portion generally designated at 384; cylindrical side portion generally designated at 386; said cylindrical side portion having an inside wall 388 and an outside wall 390. Inside wall 388 has thread means 392 for engaging thread means 354 shown in FIG. 28B. Outside wall 390 comprises distal rim 394 and proximal rim 396. This design is intended to function in a syringe for injecting a fluid into a site.

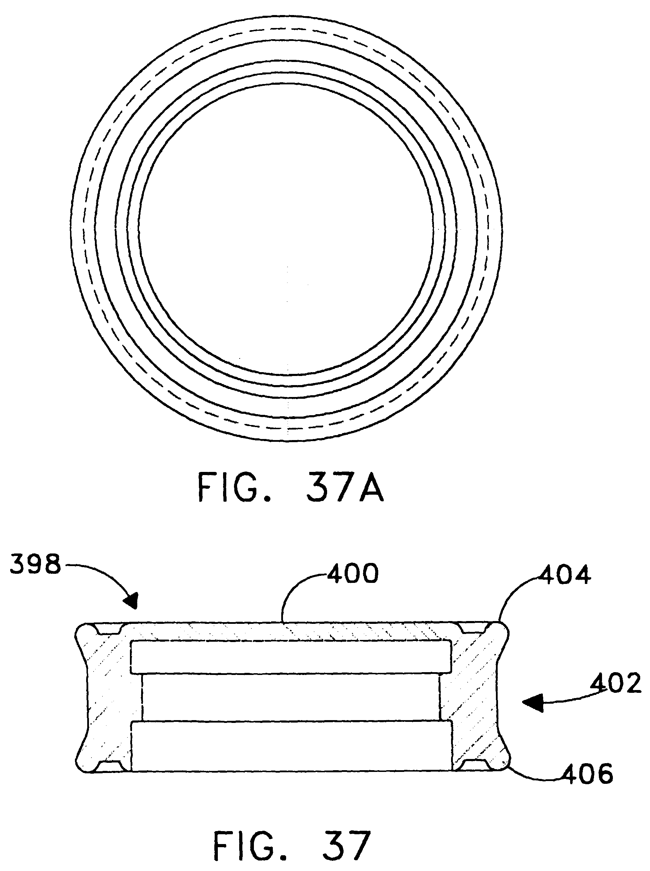

FIG. 37 and 37A show a still further embodiment of the full-face plunger ring 398 in which the top is flat instead of being a conical shape. The full-face plunger ring comprises: a horizontal or flat top portion 400 and side portion generally designated at 402. Side portion comprises distal rim 404 and proximal rim 406. The full-face plunger ring is positioned on a plunger ring tip. This design is intended for use in injecting to a site in combination with the other components of a syringe or cartridge.

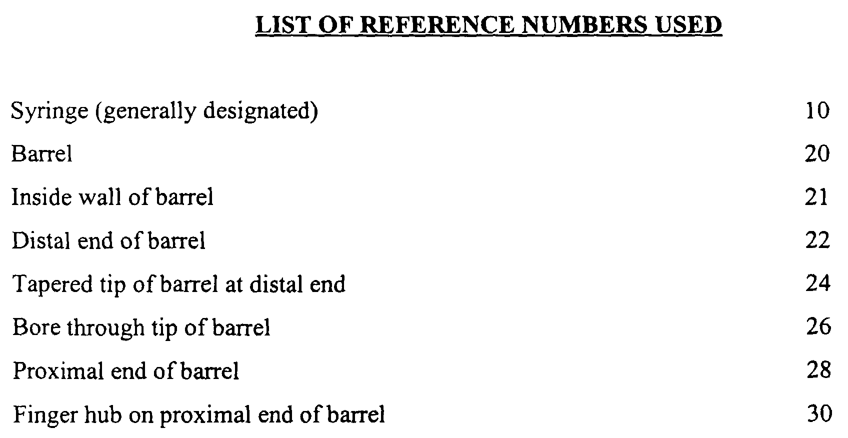

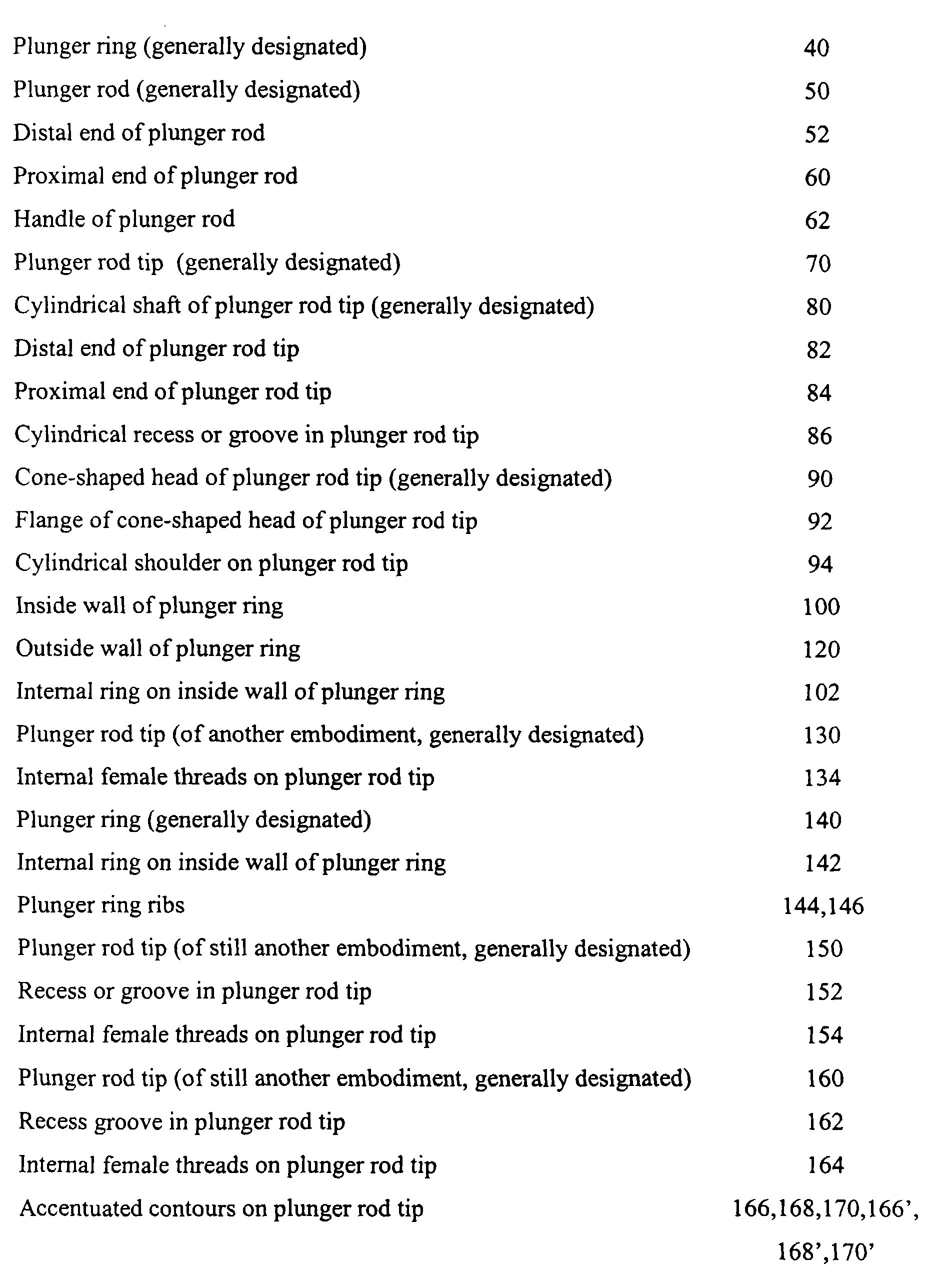

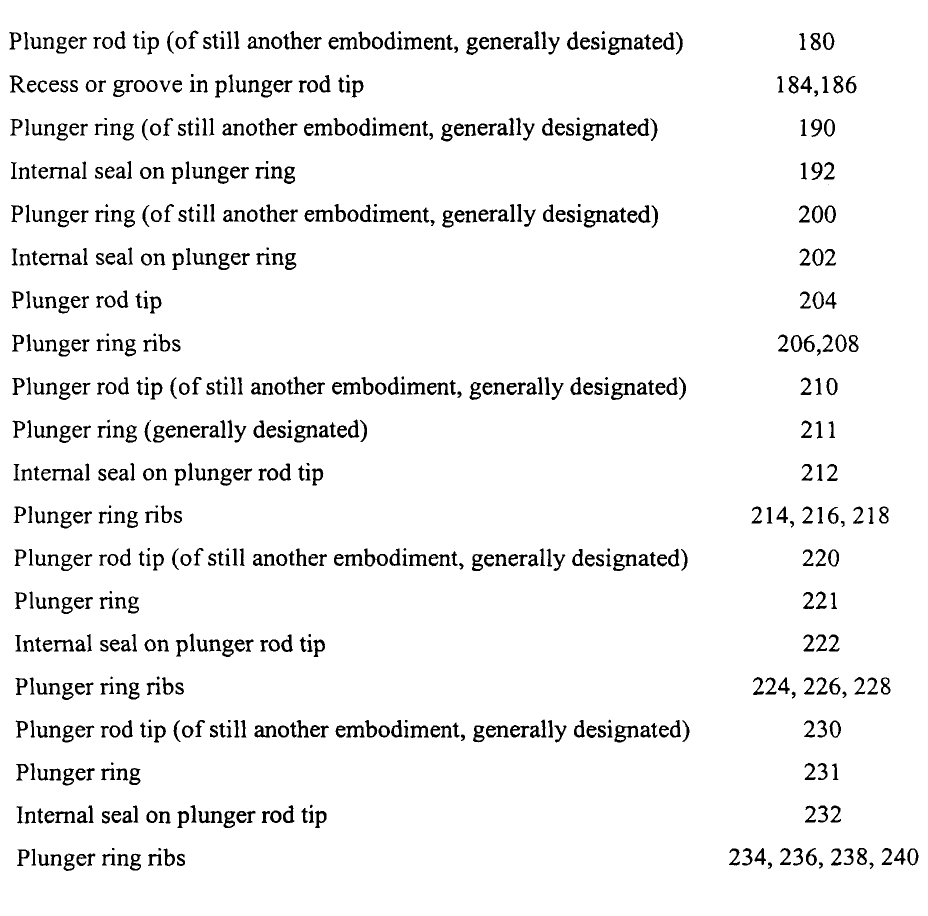

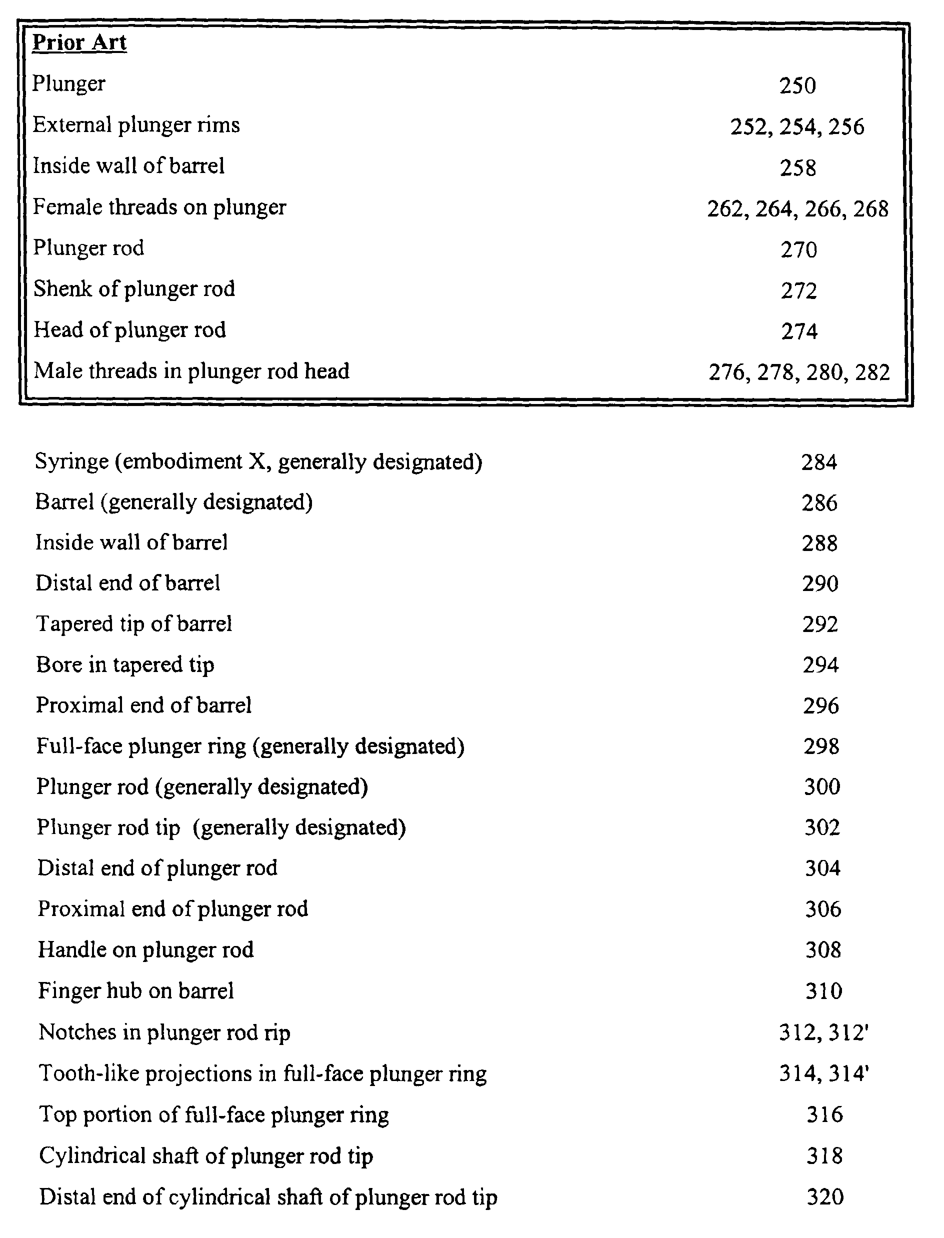

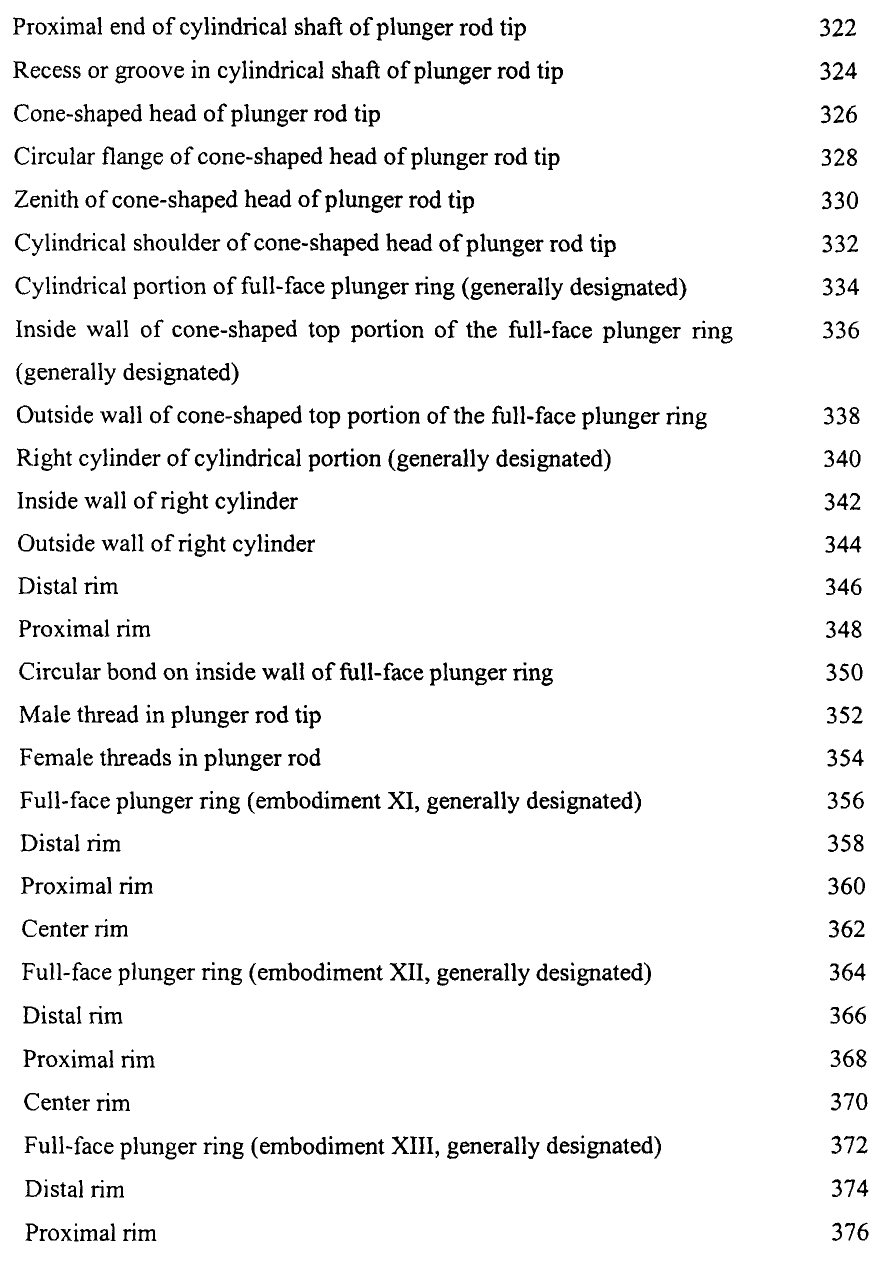

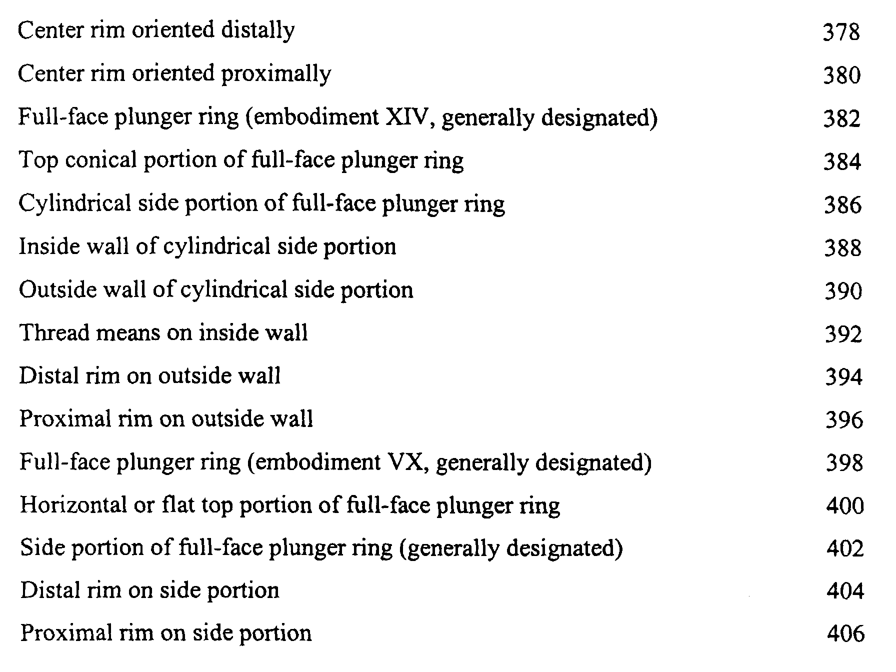

## Claims

1. An elastomeric plunger ring (298, 356, 372, 382, 398) providing a low-friction slideable sealing engagement between said elastomeric plunger ring and the inside wall (288) of a syringe or cartridge barrel (286), and interfacing and conforming to dimensional and geometric irregularities in said barrel (286), said plunger ring comprising:
a top conical or flat portion (316, 384) for interfacing a fluid contained in said syringe or cartridge barrel (286);
a cylindrical side portion (334, 386, 402) having an inside wall (342, 388) and an outside wall (344, 390);
a plurality of rims (346, 348, 358, 360, 362, 366, 368, 370, 374, 376, 378, 380, 404, 406) projecting distally, proximally or horizontally from said outside wall of said cylindrical side portion for interfacing the inside wall (288) of said syringe or cartridge barrel (286) and to form the low-friction slideable sealing engagement therewith; and receiving means on the inside wall (336) of said plunger ring to receive support means to form a non-slideable sealing engagement therewith, wherein said support means is a plunger rod tip (302) or a plunger rod tip integral with a plunger rod (300).

2. The elastomeric plunger ring of claim 1 having two rims (346, 348) on said outside wall (344) of said cylindrical side portion, one of which projects distally (346) and the other projects proximally (348) for interfacing the inside wall (288) of said syringe or cartridge barrel (286).

3. The elastomeric plunger ring of claim 1 having three rims (358, 360, 362) on said outside wall of said cylindrical side portion, two of which project distally (358, 360) and the third projects proximally (362) for interfacing the inside wall (288) of said syringe or cartridge barrel (286).

4. The elastomeric plunger ring of claim 1 having three rims (366, 368, 370) on said outside wall of said cylindrical side portion, one of which projects distally (366), another of which projects proximally (368), and the third of which projects horizontally (370).

5. The elastomeric plunger ring of claim 1 having four rims (374, 376, 378, 380) on said outside wall of said cylindrical side portion, two of which project distally (374, 378) and the other two of which project proximally (376, 380).

6. The elastomeric plunger ring of claim 1 wherein said means for receiving a support means are threads or tooth-like projections (314, 314').

7. The elastomeric plunger ring of claim 1 made of an elastomeric material selected from the group consisting of:
natural rubber;
acrylate-butadiene rubber;
cis-polybutadiene;
chlorobutyl rubber;
chlorinated polyethylene elastomers;
polyalkylene oxide polymers;
ethylene vinyl acetate;
fluorosilicone rubbers;
hexafluoropropylene-vinylidene;
tetrafluoroethylene terpolymers,
butyl rubbers;
polyisobutene;
synthetic polyisoprene rubber;
silicone rubbers;
styrene-butadiene rubbers;
tetrafluoroethylene propylene copolymers; and
thermoplastic-copolyesters.

8. A syringe (284) designed for injecting a fluid into a site or withdrawing a fluid from a site comprising:
(a) a syringe barrel (286) having an inside wall (288) defining a cylindrical chamber for retaining a fluid therein; said syringe barrel (286) having:
(1) a distal end (290) terminating in a tapered tip (292) and having a bore (294) therethrough to which an injection needle or a connector equipped with a tubing conduit can be attached; and
(2) a proximal end (296) for receiving an elastomeric plunger ring;
(b) the elastomeric plunger ring (298, 356, 372, 382, 398) according to claims 1-7.

9. The syringe of claim 8, wherein said plunger rod tip (302) having a cap portion and a cylindrical shaft portion (318), said cap portion having a plurality of notches (312, 312') therein, said cylindrical shaft (318) having a distal end (320) and a proximal end (322) and one or more recesses or grooves (324) therebetween, said plurality of notches designed to non-slideably engage projections on said inside wall (336) of said elastomeric plunger ring, and said recesses or grooves (324) to non-slideably engage said receiving means on the elastomeric plunger ring, whereby the elastomeric plunger ring is securely held on said plunger rod tip.

10. The syringe of claim 9, wherein said rod tip cylindrical shaft (318) contains male thread means (352) to receive female thread means (354) of a plunger rod (300).

11. The syringe of claims 9-10, wherein said cap portion is of cone-face or flat configuration.

12. The syringe of claims 9-11, wherein said plunger rod tip (302) is integral with a plunger rod (300).

13. A cartridge, optionally prefilled, designed for injecting a fluid into a site comprising:
(a) a cartridge barrel (286) having an inside wall (288) defining a cylindrical chamber for retaining a fluid therein; said cartridge barrel having:
(1) a distal end (290) terminating in a tapered tip (292) and having a bore (294) therethrough to which an injection needle or a connector equipped with a tubing conduit can be attached; and
(2) a proximal end (296) for receiving an elastomeric plunger ring;
(b) the elastomeric plunger ring (298, 356, 372, 382, 398) according to claims 1-7.

14. The cartridge of claim 13, wherein said plunger rod tip (302) having a cap portion and a cylindrical shaft portion (318), said cap portion having a plurality of notches (312, 312') therein, said cylindrical shaft (318) having a distal end (320) and a proximal end (322) and one or more recesses or grooves (324) therebetween, said plurality of notches designed to non-slideably engage projections on said inside wall (336) of said elastomeric plunger ring, and said recesses or grooves (324) to non-slideably engage said receiving means on the elastomeric plunger ring, whereby the elastomeric plunger ring is securely held on said inside wall of said plunger rod tip.

15. The cartridge of claim 14, wherein said rod tip cylindrical shaft (318) contains male thread means (352) to receive female thread means (354) of a plunger rod (300).

16. The cartridge of claims 14-15, wherein said cap portion is of cone-face or flat configuration.

17. The cartridge of claims 14-16, wherein said plunger rod tip (302) is integral with a plunger rod (300).

18. The cartridge of claims 13-17, wherein said cartridge barrel is made of an inert gas impermeable material selected from the group consisting of: polyethylene, polypropylene, polystyrene, acrylic polymers and methacrylic polymers.

19. The cartridge of claims 13-18, wherein said fluid is an injectable medical fluid.

20. The cartridge of claim 19, wherein said medical fluid is diagnostic contrasting media.
